(19) Europäisches Patentamt
European Patent Office
Office européen des brevets

(11) **EP 4 663 244 A2**

(12) **EUROPEAN PATENT APPLICATION**

(43) Date of publication:
**17.12.2025 Bulletin 2025/51**

(21) Application number: **25197443.2**

(22) Date of filing: **18.10.2019**

(51) International Patent Classification (IPC):
***A61P 35/00*** *(2006.01)*

(52) Cooperative Patent Classification (CPC):
**A61P 35/00; C07K 16/2803; C07K 16/2818;**
A61K 2039/507

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.10.2018 US 201862748089 P**

(62) Document number(s) of the earlier application(s) in
accordance with Art. 76 EPC:
**24182623.9 / 4 445 958**
**19798464.4 / 3 866 850**

(71) Applicant: **Bristol-Myers Squibb Company
Princeton, NJ 08543 (US)**

(72) Inventors:
• **MAURER, Matthew
Princeton, 08543 (US)**

• **SIMONSEN, Katy L
Princeton, 08543 (US)**

(74) Representative: **Mewburn Ellis LLP
Aurora Building
Counterslip
Bristol BS1 6BX (GB)**

Remarks:
•This application was filed on 21-08-2025 as a
divisional application to the application mentioned
under INID code 62
•Claims filed after the date of filing of the application
(Rule 68(4) EPC)/after the date of receipt of the
divisional application (Rule 68(4) EPC)

(54) **COMBINATION THERAPY FOR MELANOMA**

(57) The invention provides a method of treating a previously untreated metastatic or unresectable melanoma.

**EP 4 663 244 A2**

**Description**

CROSS-REFERENCE TO RELATED APPLICATIONS

This application claims priority to U.S. Provisional Application No. 62/748,089, filed October 19, 2018, which is incorporated herein by reference in its entirety.

FIELD OF THE INVENTION

The invention disclosed herein relates to methods of treating a metastatic or unresectable melanoma in a human patient with a combination of a LAG-3 inhibitor and a PD-1 pathway inhibitor.

BACKGROUND OF THE INVENTION

Lymphocyte activation gene-3 (LAG-3; CD223) is a type I transmembrane protein that is expressed on the cell surface of activated CD4+ and CD8+ T cells and subsets of NK and dendritic cells (Triebel F, et al., J. Exp. Med. 1990; 171:1393-1405; Workman C J, et al., J. Immunol. 2009; 182(4):1885-91). LAG-3 is closely related to CD4, which is a co-receptor for T helper cell activation. Both molecules have 4 extracellular Ig-like domains and bind to major histocompatibility complex (MHC) class II. In contrast to CD4, LAG-3 is only expressed on the cell surface of activated T cells and its cleavage from the cell surface terminates LAG-3 signaling. LAG-3 can also be found as a soluble protein but its function is unknown.

PD-1 is a cell surface signaling receptor that plays a critical role in the regulation of T cell activation and tolerance (Keir M E, et al., Annu Rev Immunol 2008; 26:677-704). It is a type I transmembrane protein and together with BTLA, CTLA-4, ICOS and CD28, comprise the CD28 family of T cell co-stimulatory receptors. PD-1 is primarily expressed on activated T cells, B cells, and myeloid cells (Dong H, et al., Nat Med. 1999; 5:1365-1369). It is also expressed on natural killer (NK) cells (Terme M, et al., Cancer Res 2011; 71:5393-5399). Binding of PD-1 by its ligands, PD-L1 and PD-L2, results in phosphorylation of the tyrosine residue in the proximal intracellular immune receptor tyrosine inhibitory domain, followed by recruitment of the phosphatase SHP-2, eventually resulting in down-regulation of T cell activation. One important role of PD-1 is to limit the activity of T cells in peripheral tissues at the time of an inflammatory response to infection, thus limiting the development of autoimmunity (Pardoll D M., Nat Rev Cancer 2012; 12:252-264). Evidence of this negative regulatory role comes from the finding that PD-1-deficient mice develop lupus-like autoimmune diseases including arthritis and nephritis, along with cardiomyopathy (Nishimura H, et al., Immunity, 1999; 11:141-151; and Nishimura H, et al., Science, 2001; 291:319-322). PD-1 is expressed on tumor-infiltrating lymphocytes, and its ligands are up-regulated on the cell surface of many different tumors (Dong H, et al., Nat Med 2002; 8:793-800). Multiple murine cancer models have demonstrated that binding of ligand to PD-1 results in immune evasion. In addition, blockade of this interaction results in anti-tumor activity (Topalian S L, et al. NEJM 2012; 366(26):2443-2454; Hamid O, et al., NEJM 2013; 369:134-144). Moreover, it has been shown that inhibition of the PD-1/PD-L1 interaction mediates potent antitumor activity in preclinical models (U.S. Pat. Nos. 8,008,449 and 7,943,743).

It is an object of the present invention to provide improved methods for treating metastatic or unresectable melanoma.

SUMMARY OF THE INVENTION

One aspect of the invention disclosed herein relates to a method of inhibiting the growth of a metastatic melanoma tumor in a human patient, the method comprising administering to the patient an effective amount of: (a) a LAG-3 antagonist; and (b) a PD-1 pathway inhibitor; wherein the patient has not received prior treatment for metastatic melanoma.

An additional aspect of the invention disclosed herein relates to a method of treating a metastatic melanoma tumor in a human patient, the method comprising administering to the patient an effective amount of: (a) a LAG-3 antagonist; and (b) a PD-1 pathway inhibitor; wherein the patient has not received prior systemic treatment for metastatic melanoma.

One aspect of the invention disclosed herein relates to a method of inhibiting the growth of an unresectable melanoma tumor in a human patient, the method comprising administering to the patient an effective amount of: (a) a LAG-3 antagonist; and (b) a PD-1 pathway inhibitor; wherein the patient has not received prior systemic treatment for unresectable melanoma.

Another aspect of the invention disclosed herein relates to a method of treating an unresectable melanoma tumor in a human patient, the method comprising administering to the patient an effective amount of: (a) a LAG-3 antagonist; and (b) a PD-1 pathway inhibitor; wherein the patient has not received prior systemic treatment for unresectable melanoma.

In certain embodiments, the patient received prior treatment when the melanoma was locally advanced, wherein the prior treatment was adjuvant or neoadjuvant anti-PD-1 or anti-CTLA-4 therapy. In some embodiments, the patient has histologically confirmed unresectable stage III or IV melanoma. In certain embodiments, the patient has an Eastern Cooperative Oncology Group (ECOG) performance status of 0 or 1. In some embodiments, the patient has measurable disease as determined by Response Evaluation Criteria in Solid Tumors (RECIST) version 1.1.

In certain embodiments, the patient's tumor infiltrating lymphocytes express LAG-3. In particular embodiments, greater than 1% of the patient's tumor infiltrating lymphocytes cells express LAG-3. In certain embodiments, LAG-3 expression is determined from a tumor biopsy test tissue sample. In certain embodiments, the test tissue sample is a formalin-fixed paraffin embedded (FFPE) sample. In some embodiments, LAG-3 expression is determined by measuring protein or RNA expression in the test tissue sample. In certain embodiments, LAG-3 expression is detected by an assay capable of detecting the level of LAG-3 protein in the test tissue sample. In other embodiments, LAG-3 expression is detected by an immunohistochemistry assay. In certain embodiments, the immunohistochemistry assay is a monoplex assay. In some embodiments, immunohistochemistry assay is a multiplex assay. In certain embodiments, the immunohistochemistry assay comprises contacting the tumor sample with the 17B4, SP346, 11E3, 874501, or EPR4392(2) anti-human LAG-3 monoclonal antibody.

In some embodiments, the patient's tumor cells express PD-L1. In certain embodiments, greater than 1% of the patient's tumor cells express PD-L1. In some embodiments, PD-L1 expression is determined from a tumor biopsy test tissue sample. In certain embodiments, the test tissue sample is a formalin-fixed paraffin embedded (FFPE) sample. In particular embodiments, PD-L1 expression is determined by measuring protein or RNA expression in the test tissue sample. In some embodiments, PD-L1 expression is detected by an assay capable of detecting the level of PD-L1 protein in the test tissue sample. In certain embodiments, PD-L1 expression is detected by an immunohistochemistry assay. In particular embodiments, the immunohistochemistry assay is a monoplex assay. In other embodiments, the immunohistochemistry assay is a multiplex assay.

In certain embodiments, the patient's tumor cells contain a BRAF V600 mutation.

In some embodiments, the LAG-3 antagonist is an anti-LAG-3 antibody. In certain embodiments, the anti-LAG-3 antibody is a full-length antibody. In particular embodiments, the antibody is a monoclonal, human, humanized, chimeric, or multispecific antibody. In certain embodiments, the multispecific antibody is a dual-affinity re-targeting antibody (DART), a DVD-Ig, or bispecific antibody. In some embodiments, the antibody is a F(ab')2 fragment, a Fab' fragment, a Fab fragment, a Fv fragment, a scFv fragment, a dsFv fragment, a dAb fragment, or a single chain binding polypeptide. In some embodiments, the anti-LAG-3 antibody is BMS-986016, IMP731 (H5L7BW), MK-4280 (28G-10), REGN3767, GSK2831781, humanized BAP050, IMP-701 (LAG-525), aLAG3(0414), aLAG3(0416), Sym022, TSR-033, TSR-075, XmAb22841, MGD013, BI754111, FS118, P 13B02-30, or AVA-017. In particular embodiments, the LAG-3 antagonist is IMP321.

In some embodiments, the anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5. In some embodiments, the anti-LAG-3 antibody comprises (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO:11; and (f) a light chain variable region CDR3 comprising the sequence set forth in SEQ ID NO:12. In certain embodiments, the anti-LAG-3 antibody comprises heavy and light chain variable regions comprising the sequences set forth in SEQ ID NOs:3 and 5, respectively. In particular embodiments, the anti-LAG-3 antibody comprises heavy and light chains comprising the sequences set forth in SEQ ID NOs: 1 and 2, respectively.

In some embodiments, the PD-1 pathway inhibitor is an anti-PD-1 or an anti-PD-L1 antibody. In certain embodiments, the anti-PD-1 antibody is selected from the group consisting of: nivolumab, pembrolizumab, pidilizumab, PDR001, MEDI0680, TSR-042, REGN2810, JS001, PF-06801591, BGB-A317, BI 754091, and SHR-1210. In certain embodiments, the anti-PD-1 antibody comprises CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17. In particular embodiments, the anti-PD-1 antibody comprises (a) a heavy chain variable region CDR1 comprising the sequence set forth in SEQ ID NO:19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24. In some embodiments, the anti-PD-1 antibody comprises heavy and light chain variable regions comprising the sequences set forth in SEQ ID NOs: 15 and 17, respectively. In certain embodiments, the anti-PD-1 antibody comprises heavy and light chains comprising the sequences as set forth in SEQ ID NOs: 13 and 14, respectively.

In some embodiments, a fixed dose combination of the anti-LAG-3 or LAG-3 antagonist and anti-PD-1 antibody are administered. In certain embodiments, the method comprises at least one administration cycle, wherein the cycle is a period of four weeks, and wherein for each of the at least one cycle, one dose of the anti-LAG-3 antibody or antagonist is administered at a dose of 160 mg and one dose of the anti-PD-1 antibody are administered at a dose of 480 mg.

In certain embodiments, the anti-LAG-3 antibody or antagonist and anti-PD-1 antibody are formulated for intravenous administration. In some embodiments, the anti-LAG-3 antibody or antagonist and anti-PD-1 antibody are formulated together. In embodiments, the anti-LAG-3 antibody or antagonist and anti-PD-1 antibody are formulated separately.

One aspect of the invention disclosed herein relates to a method of inhibiting the growth of a metastatic melanoma tumor in a human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma.

One aspect of the invention disclosed herein relates to a method of treating metastatic melanoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma.

One aspect of the invention disclosed herein relates to a method of inhibiting the growth of an unresectable melanoma tumor in a human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma.

Another aspect of the invention disclosed herein relates to a method of treating unresectable melanoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma.

One aspect of the invention disclosed herein relates to a method of inhibiting the growth of a metastatic melanoma tumor in a human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma; and wherein at least one dose of the anti-LAG-3 antibody is administered at a dose of 160 mg and at least one dose of the anti-PD-1 antibody are administered at a dose of 480 mg every four weeks.

An aspect of the invention disclosed herein relates to a method of treating metastatic melanoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma; and wherein at least one dose of the anti-LAG-3 antibody is administered at a dose of 160 mg and at least one dose of the anti-PD-1 antibody is administered at a dose of 480 mg every four weeks.

One aspect of the invention disclosed herein relates to a method of inhibiting the growth of an unresectable melanoma tumor in a human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

An additional aspect of the invention disclosed herein relates to a method of treating unresectable melanoma in a human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

One aspect of the invention disclosed herein relates to a method of inhibiting the growth of a metastatic melanoma tumor in an adolescent human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma; and wherein at least one dose of the anti-LAG-3 antibody is administered at a dose of 2 mg/kg and at least one dose of the anti-PD-1 antibody is administered at a dose of 6 mg/kg every four weeks.

One aspect of the invention disclosed herein relates to a method of treating metastatic melanoma in an adolescent human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma; and wherein at least one dose of the anti-LAG-3 antibody is administered at a dose of 2 mg/kg and at least one dose of the anti-PD-1 antibody is administered at a dose of 6 mg/kg every four weeks.

One aspect of the invention disclosed herein relates to a method of inhibiting the growth of an unresectable melanoma tumor in an adolescent human patient, the method comprising administering to the patient an effective amount of each of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma; and wherein at least one dose of the anti-LAG-3 antibody is administered at a dose of 2 mg/kg and at least one dose of the anti-PD-1 antibody is administered at a dose of 6 mg/kg every four weeks.

Another aspect of the invention disclosed herein relates to a method of treating unresectable melanoma in an adolescent human patient, the method comprising administering to the patient an effective amount of: (a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma; and wherein at least one dose of the anti-LAG-3 antibody is administered at a dose of 2 mg/kg and at least one dose of the anti-PD-1 antibody is administered at a dose of 6 mg/kg every four weeks.

In some embodiments, the patient is 12 or more years of age. In certain embodiments, the patient is between 12 and 17 years of age. In particular embodiments, the prior system treatment is adjuvant or neoadjuvant anti-PD-1 or anti-CTLA-4 therapy. In other embodiments, the patient has histologically confirmed unresectable stage III or IV melanoma. In certain embodiments, the patient has an Eastern Cooperative Oncology Group (ECOG) performance status of 0 or 1 and/or a Lansky performance score of 80% or greater. In some embodiments, the patient has measurable disease as determined by Response Evaluation Criteria in Solid Tumors (RECIST) version 1.1.

In some embodiments, the patient's tumor infiltrating lymphocytes cells express LAG-3. In certain embodiments, greater than 1% of the patient's tumor 71. In certain embodiments, LAG-3 expression is determined from a tumor biopsy test tissue sample. In one embodiment, the test tissue sample is a formalin-fixed paraffin embedded (FFPE) sample. In certain embodiments, LAG-3 expression is determined by measuring protein or RNA expression in the test tissue sample. In some embodiments, LAG-3 expression is detected by an assay capable of detecting the level of LAG-3 protein in the test tissue sample. In certain embodiments, LAG-3 expression is detected by an immunohistochemistry assay. In some embodiments, the immunohistochemistry assay is a monoplex assay. In other embodiments, the immunohistochemistry assay is a multiplex assay. In particular embodiments, the immunohistochemistry assay comprises contacting the tumor sample with the 17B4, SP346, 11E3, 874501, or EPR4392(2) anti-human LAG-3 monoclonal antibody associated tumor infiltrating lymphocytes express LAG-3.

In an embodiment, the patient's tumor cells express PD-L1. In certain embodiments, greater than 1% of the patient's tumor cells express PD-L1. In some embodiments, PD-L1 expression is determined from a tumor biopsy test tissue sample. In certain embodiments, the test tissue sample is a formalin-fixed paraffin embedded (FFPE) sample. In particular embodiments, PD-L1 expression is determined by measuring protein or RNA expression in the test tissue sample. In some embodiments, PD-L1 expression is detected by an assay capable of detecting the level of PD-L1 protein in the test tissue sample. In certain embodiments, PD-L1 expression is detected by an immunohistochemistry assay. In particular embodiments, the immunohistochemistry assay is a monoplex assay. In certain embodiments, the immunohistochemistry assay is a multiplex assay. In some embodiments, the patient's tumor cells contain a BRAF V600 mutation.

One aspect of the invention disclosed herein relates to a method of inhibiting the growth of a metastatic melanoma tumor in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO: 17; wherein the patient has not received prior systemic treatment for metastatic melanoma.

One aspect of the invention disclosed herein relates to a method of treating metastatic melanoma in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma.

An aspect of the invention disclosed herein relates to a method of inhibiting the growth of an unresectable melanoma tumor in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma.

One aspect of the invention disclosed herein relates to a method of treating unresectable melanoma in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma.

Another aspect of the invention disclosed herein relates to a method of inhibiting the growth of a metastatic melanoma tumor in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma; and wherein at least one dose of the anti-LAG-3 antibody is administered at a dose of 160 mg and at least one dose of the anti-PD-1 antibody are administered at a dose of 480 mg every four weeks.

One aspect of the invention disclosed herein relates to a method of treating metastatic melanoma in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for metastatic melanoma; and wherein at least one dose of the anti-LAG-3 antibody is administered at a dose of 160 mg and at least one dose of the anti-PD-1 antibody is administered at a dose of 480 mg every four weeks.

Another aspect of the invention disclosed herein relates to a method of inhibiting the growth of an unresectable melanoma tumor in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

An aspect of the invention disclosed herein relates to a method of treating unresectable melanoma in a human patient, the method comprising: (a) determining expression of LAG-3 on the patient's tumor infiltrating lymphocytes, PD-L1 expression on the patient's tumor cells, and/or determining the presence of a BRAF V600 mutation in the patient's tumor cells; and (b) administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

One aspect of the invention disclosed herein relates to a method of treating unresectable melanoma in a human patient, the method comprising administering to the patient an effective amount of each of: (i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and (ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17; wherein the patient has not received prior treatment for unresectable melanoma; and wherein at least one dose of the anti-LAG-3 antibody is administered at a dose of 160 mg and at least one dose of the anti-PD-1 antibody is administered at a dose of 480 mg every four weeks. In some embodiments, the anti-LAG-3 antibody and the anti-PD-1 antibody are a first-line treatment.

One aspect of the invention disclosed herein relates to a method of treating unresectable melanoma in a human patient, the method comprising administering to the patient an effective amount of each of: (i) an anti-LAG-3 antibody comprising (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; and (e) a light chain variable region CDR2 comprising the sequence set forth in SEQ ID NO:11, and (ii) an anti-PD-1 antibody comprising (a) a heavy chain variable region CDR1 comprising the sequence set forth in SEQ ID NO:19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24, wherein the patient has not received prior treatment for unresectable melanoma; and wherein at least one dose of the anti-LAG-3 antibody is administered at a dose of 160 mg and at least one dose of the anti-PD-1 antibody is administered at a dose of 480 mg every four weeks. In certain embodiments, the the anti-LAG-3 antibody and the anti-PD-1 antibody are administered as a first-line treatment.

In some embodiments herein, the anti-LAG-3 antibody comprises heavy and light chains comprising the sequences set forth in SEQ ID NOs: 30 and 2, respectively.

One aspect of the invention disclosed herein relates to a method of treating unresectable melanoma in a human patient, the method comprising administering to the patient an effective amount of each of relatlimab and nivolumab, wherein the patient has not received prior treatment for unresectable melanoma; and wherein at least one dose of relatlimab is administered at a dose of 160 mg and at least one dose of nivolumab is administered at a dose of 480 mg every four weeks.

One aspect of the invention disclosed herein relates to a method of treating unresectable melanoma in a human patient, the method comprising administering to the patient an effective amount of each of: (i) an anti-LAG-3 antibody comprising heavy and light chains comprising the sequences set forth in SEQ ID NOs: 30 and 2, respectively, and (ii) an anti-PD-1 antibody comprising the sequences as set forth in SEQ ID NOs: 13 and 14, respectively, wherein the patient has not received prior treatment for unresectable melanoma; and wherein at least one dose of the anti-LAG-3 antibody is administered at a dose of 160 mg and at least one dose of the anti-PD-1 antibody is administered at a dose of 480 mg every four weeks.

One aspect of the invention disclosed herein relates to a method of treating metastatic melanoma in a human patient, the method comprising administering to the patient an effective amount of each of relatlimab and nivolumab, wherein the patient has not received prior treatment for metastatic melanoma; and wherein at least one dose of the relatlimab is administered at a dose of 160 mg and at least one dose of the nivolumab is administered at a dose of 480 mg every four weeks.

One aspect of the invention disclosed herein relates to a method of treating metastatic melanoma in a human patient, the method comprising administering to the patient an effective amount of each of: (i) an anti-LAG-3 antibody comprising heavy and light chains comprising the sequences set forth in SEQ ID NOs: 30 and 2, respectively, and (ii) an anti-PD-1 antibody comprising the sequences as set forth in SEQ ID NOs: 13 and 14, respectively, wherein the patient has not received prior treatment for metastatic melanoma; and wherein at least one dose of the anti-LAG-3 antibody is administered at a dose of 160 mg and at least one dose of the anti-PD-1 antibody is administered at a dose of 480 mg every four weeks.

One aspect of the invention disclosed herein relates to a method of treating metastatic or unresectable melanoma in a human patient, the method comprising administering to the patient an effective amount of each of relatlimab and nivolumab, wherein the patient has not received prior treatment for unresectable melanoma; and wherein at least one dose of the relatlimab is administered at a dose of 160 mg and at least one dose of the nivolumab is administered at a dose of 480 mg every four weeks. One aspect of the invention disclosed herein relates to a method of treating metastatic or unresectable melanoma in a human patient, the method comprising administering to the patient an effective amount of each of: (i) an anti-LAG-3 antibody comprising heavy and light chains comprising the sequences set forth in SEQ ID NOs: 30 and 2, respectively, and (ii) an anti-PD-1 antibody comprising the sequences as set forth in SEQ ID NOs: 13 and 14, respectively, wherein the patient has not received prior treatment for metastatic or unresectable melanoma; and wherein at least one dose of the anti-LAG-3 antibody is administered at a dose of 160 mg and at least one dose of the anti-PD-1 antibody is administered at a dose of 480 mg every four weeks.

One aspect of the invention disclosed herein relates to a product comprising relatlimab and nivolumab for combined use in treating metastatic or unresectable melanoma in a human patient that has not received prior treatment for metastatic or unresectable melanoma; wherein the treatment comprises administering at least one dose of relatlimab at a dose of 160 mg and at least one dose of nivolumab at a dose of 480 mg every four weeks.

One aspect of the invention disclosed herein relates to a product comprising an anti-LAG-3 antibody comprising heavy and light chains comprising the sequences set forth in SEQ ID NOs: 30 and 2, respectively and an anti-PD-1 antibody comprising the sequences as set forth in SEQ ID NOs: 13 and 14, respectively for combined use in treating metastatic or unresectable melanoma in a human patient that has not received prior treatment for metastatic or unresectable melanoma; wherein the treatment comprises administering at least one dose of the anti-LAG-3 antibody at a dose of 160 mg and at least one dose of the anti-PD-1 antibody at a dose of 480 mg every four weeks.

One aspect of the invention disclosed herein relates to a product comprising an anti-LAG-3 antibody comprising (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO:11, and an anti-PD-1 antibody comprising (a) a heavy chain variable region CDR1 comprising the sequence set forth in SEQ ID NO:19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24, for combined use in treating metastatic or unresectable melanoma in a human patient that has not received prior treatment for metastatic or unresectable melanoma; wherein the treatment comprises administering at least one dose of the anti-LAG-3 antibody at a dose of 160 mg and at least one dose of the anti-PD-1 antibody at a dose of 480 mg every four weeks.

## DETAILED DESCRIPTION OF THE INVENTION

In one aspect, the present invention relates to an improved method of treatment for malignant tumors in a human patient. In particular, the present invention shows that the administration of an anti-LAG-3 antibody in combination with an anti-PD-1 antibody, achieves surprisingly improved treatment outcomes in a patient population not having received prior systemic therapy. Accordingly, in one aspect, the invention described herein relates to a method of treating previously untreated metastatic or unresectable melanoma by administering a combination of a LAG-3 inhibitor (e.g., anti-LAG-3 antibody) and a PD-1 pathway inhibitor (e.g., an anti-PD-1 antibody).

### 1. Definitions

In order that the present disclosure may be more readily understood, certain terms are first defined. As used in this application, except as otherwise expressly provided herein, each of the following terms shall have the meaning set forth below. Additional definitions are set forth throughout the application.

An "antibody" (Ab) shall include, without limitation, a glycoprotein immunoglobulin which binds specifically to an antigen and comprises at least two heavy (H) chains and two light (L) chains interconnected by disulfide bonds. Each H chain comprises a heavy chain variable region (abbreviated herein as $V_H$) and a heavy chain constant region. The heavy chain constant region comprises three constant domains, $C_{H1}$, $C_{H2}$ and $C_{H3}$. Each light chain comprises a light chain variable region (abbreviated herein as $V_L$) and a light chain constant region. The light chain constant region is comprises one constant domain, $C_L$. The $V_H$ and $V_L$ regions can be further subdivided into regions of hypervariability, termed complementarity determining regions (CDRs), interspersed with regions that are more conserved, termed framework regions (FR). Each $V_H$ and $V_L$ comprises three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. A heavy chain may have the C-terminal lysine or not. Unless specified otherwise herein, the amino acids in the variable regions are numbered using the Kabat numbering system and those in the constant regions are numbered using the EU system. In one embodiment, an antibody is a full-length antibody.

An immunoglobulin may derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the antibody class or subclass (e.g., IgM or IgG1) that is encoded by the heavy chain constant region genes. The term "antibody" includes, by way of example, monoclonal and polyclonal antibodies; chimeric and humanized antibodies; human or nonhuman antibodies; wholly synthetic antibodies; and single chain antibodies. A nonhuman antibody may be humanized by recombinant methods to reduce its immunogenicity in man. Where not expressly stated, and unless the context indicates otherwise, the term "antibody" includes monospecific, bispecific, or multi-specific antibodies, as well as a single chain antibody. In some embodiments, the antibody is a bispecific antibody. In other embodiments, the antibody is a monospecific antibody. In one aspect, the constant region isotype is IgG4 with a mutation at amino acid residues 228, e.g., S228P.

As used herein, an "IgG antibody" has the structure of a naturally occurring IgG antibody, *i.e.,* it has the same number of heavy and light chains and disulfide bonds as a naturally occurring IgG antibody of the same subclass. For example, an anti-LAG-3 IgG1, IgG2, IgG3 or IgG4 antibody consists of two heavy chains (HCs) and two light chains (LCs), wherein the two heavy chains and light chains are linked by the same number and location of disulfide bridges that occur in native IgG1, IgG2, IgG3 and IgG4 antibodies, respectively (unless the antibody has been mutated to modify the disulfide bonds)

An "isolated antibody" refers to an antibody that is substantially free of other antibodies having different antigenic specificities (*e.g.*, an isolated antibody that binds specifically to LAG-3 is substantially free of antibodies that do not bind specifically to LAG-3). An isolated antibody that binds specifically to LAG-3 may, however, have cross-reactivity to other antigens, such as LAG-3 molecules from different species. Moreover, an isolated antibody may be substantially free of other cellular material and/or chemicals.

The antibody may be an antibody that has been altered (*e.g.*, by mutation, deletion, substitution, conjugation to a non-antibody moiety). For example, an antibody may include one or more variant amino acids (compared to a naturally occurring antibody) which change a property (*e.g.*, a functional property) of the antibody. For example, numerous such alterations are known in the art which affect, *e.g.*, half-life, effector function, and/or immune responses to the antibody in a patient. The term antibody also includes artificial polypeptide constructs which comprise at least one antibody-derived antigen binding site.

The term "monoclonal antibody" ("mAb") refers to a non-naturally occurring preparation of antibody molecules of single molecular composition, *i.e.*, antibody molecules whose primary sequences are essentially identical, and which exhibits a single binding specificity and affinity for a particular epitope. A mAb is an example of an isolated antibody. MAbs may be produced by hybridoma, recombinant, transgenic or other techniques known to those skilled in the art.

A "human" antibody (HuMAb) refers to an antibody having variable regions in which both the framework and CDR regions are derived from human germline immunoglobulin sequences. Furthermore, if the antibody contains a constant region, the constant region is also derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*). However, the term "human antibody," as used herein, is not intended to include antibodies in which CDR sequences derived from the germline of another mammalian species, such as a mouse, have been grafted onto human framework sequences. The terms "human" antibodies and "fully human" antibodies and are used synonymously.

A "humanized antibody" refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

An "anti-antigen" antibody refers to an antibody that binds specifically to the antigen. For example, an anti-LAG-3 antibody binds specifically to LAG-3.

An "antigen-binding portion" of an antibody (also called an "antigen-binding fragment") refers to one or more fragments of an antibody that retain the ability to bind specifically to the antigen bound by the whole antibody. It has been shown that the antigen-binding function of an antibody can be performed by fragments or portions of a full-length antibody. Examples of binding fragments encompassed within the term "antigen-binding portion" or "antigen-binding fragment" of an antibody, e.g., an anti-LAG-3 antibody described herein, include:

(1) a Fab fragment (fragment from papain cleavage) or a similar monovalent fragment consisting of the VL, VH, LC and CH1 domains;
(2) a F(ab')2 fragment (fragment from pepsin cleavage) or a similar bivalent fragment comprising two Fab fragments linked by a disulfide bridge at the hinge region;
(3) a Fd fragment consisting of the VH and CH1 domains;
(4) a Fv fragment consisting of the VL and VH domains of a single arm of an antibody,
(5) a single domain antibody (dAb) fragment (Ward et al., (1989) Nature 341:544-46), which consists of a VH domain;
(6) a bi-single domain antibody which consists of two VH domains linked by a hinge (dual-affinity re-targeting antibodies (DARTs));
(7) a dual variable domain immunoglobulin;
(8) an isolated complementarity determining region (CDR); and
(9) a combination of two or more isolated CDRs, which can optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); *see, e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" or "antigen-binding fragment" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins. In some embodiments, an antibody is an antigen-binding fragment.

The term "LAG-3" refers to Lymphocyte Activation Gene-3. The term "LAG-3" includes variants, isoforms, homologs, orthologs and paralogs. For example, antibodies specific for a human LAG-3 protein may, in certain cases, cross-react with a LAG-3 protein from a species other than human. In other embodiments, the antibodies specific for a human LAG-3 protein may be completely specific for the human LAG-3 protein and may not exhibit species or other types of cross-reactivity, or may cross-react with LAG-3 from certain other species, but not all other species (e.g., cross-react with monkey LAG-3 but not mouse LAG-3). The term "human LAG-3" refers to human sequence LAG-3, such as the complete amino acid sequence of human LAG-3 having GenBank Accession No. NP_002277. The term "mouse LAG-3" refers to mouse sequence LAG-3, such as the complete amino acid sequence of mouse LAG-3 having GenBank Accession No. NP_032505. LAG-3 is also known in the art as, for example, CD223. The human LAG-3 sequence may differ from human LAG-3 of GenBank Accession No. NP_002277 by having, e.g., conserved mutations or mutations in non-conserved regions and the LAG-3 has substantially the same biological function as the human LAG-3 of GenBank Accession No. NP_002277. For example, a biological function of human LAG-3 is having an epitope in the extracellular domain of LAG-3 that is specifically bound by an antibody of the instant disclosure or a biological function of human LAG-3 is binding to MHC Class II molecules.

A particular human LAG-3 sequence will generally be at least 90% identical in amino acid sequence to human LAG-3 of GenBank Accession No. NP_002277 and contains amino acid residues that identify the amino acid sequence as being human when compared to LAG-3 amino acid sequences of other species (e.g., murine). In certain cases, a human LAG-3 can be at least about 95%, or even at least about 96%, about 97%, about 98%, or about 99% identical in amino acid sequence to LAG-3 of GenBank Accession No. NP_002277. In certain embodiments, a human LAG-3 sequence will display no more than 10 amino acid differences from the LAG-3 sequence of GenBank Accession No. NP_002277. In certain embodiments, the human LAG-3 can display no more than 5, or even no more than 4, 3, 2, or 1 amino acid difference from the LAG-3 sequence of GenBank Accession No. NP_002277. Percent identity can be determined as described herein.

As used herein, the terms "Programmed Death 1," "Programmed Cell Death 1," "Protein PD-1," "PD-1," "PD1," "PDCD1," "hPD-1" and "hPD-I" are used interchangeably, and include variants, isoforms, species homologs of human PD-1, and analogs having at least one common epitope with PD-1. The complete PD-1 sequence can be found under GenBank Accession No. U64863.

The protein Programmed Death 1 (PD-1) is an inhibitory member of the CD28 family of receptors, that also includes CD28, CTLA-4, ICOS and BTLA. PD-1 is expressed on activated B cells, T cells, and myeloid cells (Agata et al., supra; Okazaki et al. (2002) Curr. Opin. Immunol. 14: 391779-82; Bennett et al. (2003) J Immunol 170:711-8). The initial members of the family, CD28 and ICOS, were discovered by functional effects on augmenting T cell proliferation following the addition of monoclonal antibodies (Hutloff et al. Nature (1999); 397:263-266; Hansen et al. Immunogenics (1980); 10:247-260). PD-1 was discovered through screening for differential expression in apoptotic cells (Ishida et al. EMBO J (1992); 11:3887-95). The other members of the family, CTLA-4 and BTLA, were discovered through screening for differential expression in cytotoxic T lymphocytes and TH1 cells, respectively. CD28, ICOS and CTLA-4 all have an unpaired cysteine residue allowing for homodimerization. In contrast, PD-1 is suggested to exist as a monomer, lacking the unpaired cysteine residue characteristic in other CD28 family members.

The PD-1 gene is a 55 kDa type I transmembrane protein that is part of the Ig gene superfamily (Agata et al. (1996) Int Immunol 8:765-72). PD-1 contains a membrane proximal immunoreceptor tyrosine inhibitory motif (ITIM) and a membrane distal tyrosine-based switch motif (ITSM) (Thomas, M. L. (1995) J Exp Med 181:1953-6; Vivier, E and Daeron, M (1997) Immunol Today 18:286-91). Although structurally similar to CTLA-4, PD-1 lacks the MYPPPY motif (SEQ ID NO: 32) that is critical for B7-1 and B7-2 binding. Two ligands for PD-1 have been identified, PD-L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1, and the effect is additive when the interaction of PD-1 with PD-L2 is blocked as well (Iwai et al. (2002) Proc. Nat'1. Acad. Sci. USA 99:12293-7; Brown et al. (2003) J. Immunol. 170:1257-66).

Consistent with PD-1 being an inhibitory member of the CD28 family, PD-1 deficient animals develop various autoimmune phenotypes, including autoimmune cardiomyopathy and a lupus-like syndrome with arthritis and nephritis (Nishimura et al. (1999) Immunity 11:141-51; Nishimura et al. (2001) Science 291:319-22). Additionally, PD-1 has been found to play a role in autoimmune encephalomyelitis, systemic lupus erythematosus, graft-versus-host disease (GVHD), type I diabetes, and rheumatoid arthritis (Salama et al. (2003) J Exp Med 198:71-78; Prokunina and Alarcon-Riquelme (2004) Hum Mol Genet 13:R143; Nielsen et al. (2004) Lupus 13:510). In a murine B cell tumor line, the ITSM of PD-1 was shown to be essential to block BCR-mediated Ca.sup.2+-flux and tyrosine phosphorylation of downstream effector molecules (Okazaki et al. (2001) PNAS 98:13866-71).

"Programmed Death Ligand-1 (PD-L1)" is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and 5 analogs having at least one common epitope with hPD-L1. The complete hPD-L1 sequence can be found under GenBank Accession No. Q9NZQ7.

The terms "Programmed Death Ligand-2" and "PD-L2" as used herein include human PD-L2 (hPD-L2), variants, isoforms, and species homologs of hPD-L2, and analogs having at least one common epitope with hPD-L2. The complete hPD-L2 sequence can be found under GenBank Accession No. Q9BQ51.

A "patient" as used herein includes any patient who is afflicted with a cancer (e.g., metastatic or unresectable melanoma). The terms "subject" and "patient" are used interchangeably herein.

"Administering" refers to the physical introduction of a composition comprising a therapeutic agent to a subject, using any of the various methods and delivery systems known to those skilled in the art. Routes of administration for the formulations disclosed herein include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. In some embodiments, the formulation is administered via a non-parenteral route, in some embodiments, orally. Other non-parenteral routes include a topical, epidermal or mucosal route of administration, for example, intranasally, vaginally, rectally, sublingually or topically. Administering can also be performed, for example, once, a plurality of times, and/or over one or more extended periods.

"Treatment" or "therapy" of a subject refers to any type of intervention or process performed on, or the administration of an active agent to, the subject with the objective of reversing, alleviating, ameliorating, inhibiting, slowing down progression, development, severity or recurrence of a symptom, complication or condition, or biochemical indicia associated with a disease. Response Evaluation Criteria In Solid Tumors (RECIST) is a measure for treatment efficacy and are established rules that define when tumors respond, stabilize, or progress during treatment. RECIST 1.1 is the current guideline to solid tumor measurement and definitions for objective assessment of change in tumor size for use in adult and pediatric cancer clinical trials. Eastern Cooperative Oncology Group (ECOG) Performance Status is a numbering scale used to define the population of patients to be studied in a trial, so that it can be uniformly reproduced among physicians who enroll patients. In pediatric patients, the Lansky Performance Scale is a method for describing functional status in children. It was derived and internally validated in children with cancer to assess response to therapies and overall status.

As used herein, "effective treatment" refers to treatment producing a beneficial effect, e.g., amelioration of at least one symptom of a disease or disorder. A beneficial effect can take the form of an improvement over baseline, i.e., an improvement over a measurement or observation made prior to initiation of therapy according to the method. A beneficial effect can also take the form of arresting, slowing, retarding, or stabilizing of a deleterious progression of a marker of solid tumor. Effective treatment may refer to alleviation of at least one symptom of a solid tumor. Such effective treatment may, e.g., reduce patient pain, reduce the size and/or number of lesions, may reduce or prevent metastasis of a tumor, and/or may slow tumor growth.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In reference to solid tumors, an effective amount comprises an amount sufficient to cause a tumor to shrink and/or to decrease the growth rate of the tumor (such as to suppress tumor growth) or to delay other unwanted cell proliferation. In some embodiments, an effective amount is an amount sufficient to prevent or delay tumor recurrence. An effective amount can be administered in one or more administrations. The effective amount of the drug or composition may: (i) reduce the number of cancer cells; (ii) reduce tumor size; (iii) inhibit, retard, slow to some extent and may stop cancer cell infiltration into peripheral organs; (iv) inhibit (i.e., slow to some extent and may stop tumor metastasis; (v) inhibit tumor growth; (vi) prevent or delay occurrence and/or recurrence of tumor; and/or (vii) relieve to some extent one or more of the symptoms associated with the cancer. In one example, an "effective amount" is the amount of anti-LAG-3 antibody and the amount of anti-PD-1 antibody, in combination, clinically proven to affect a significant decrease in cancer or slowing of progression of cancer, such as an advanced solid tumor. As used herein, the terms "fixed dose", "flat dose" and "flat-fixed dose" are used interchangeably and refer to a dose that is administered to a patient without regard for the weight or body surface area (BSA) of the patient. The fixed or flat dose is therefore not provided as a mg/kg dose, but rather as an absolute amount of the agent (e.g., the anti-LAG-3 antibody and anti-PD-1 antibody). For example, a 60 kg person and a 100 kg person would receive the same dose of the composition (e.g., 480 mg of an anti-PD-1 antibody and 160 mg of an anti- LAG-3 antibody in a single fixed dosing formulation vial).

The use of the term "fixed dose combination" with regard to a composition of the invention means that two or more different antibodies in a single composition are present in the composition in particular (fixed) ratios with each other. In some embodiments, the fixed dose is based on the weight (*e.g.*, mg) of the antibodies. In certain embodiments, the fixed dose is based on the concentration (*e.g.*, mg/ml) of the antibodies. In some embodiments, the ratio is at least about 1:1, about 1:2, about 1:3, about 1:4, about 1:5, about 1:6, about 1:7, about 1:8, about 1:9, about 1:10, about 1:15, about 1:20, about 1:30, about 1:40, about 1:50, about 1:60, about 1:70, about 1:80, about 1:90, about 1:100, about 1:120, about 1:140, about 1:160, about 1:180, about 1:200, about 200:1, about 180:1, about 160:1, about 140:1, about 120:1, about 100:1, about 90:1, about 80:1, about 70:1, about 60:1, about 50:1, about 40:1, about 30:1, about 20:1, about 15:1, about 10:1, about 9:1, about 8:1, about 7:1, about 6:1, about 5:1, about 4:1, about 3:1, or about 2:1 mg first antibody to mg second antibody. For example, the 3:1 ratio of a first antibody and a second antibody can mean that a vial can contain about 240 mg of the first antibody and 80 mg of the second antibody or about 3 mg/ml of the first antibody and 1 mg/ml of the second antibody.

The term "weight based dose" as referred to herein means that a dose that is administered to a patient is calculated based on the weight of the patient. For example, when a patient with 60 kg body weight requires 3 mg/kg of an anti-LAG-3 antibody in combination with 3 mg/kg of an anti-PD-1 antibody, one can draw the appropriate amounts of the anti-LAG-3 antibody (*i.e.,* 180 mg) and the anti-PD-1 antibody (*i.e., 180* mg) at once from a 1:1 ratio fixed dose combination of an anti-LAG3 antibody and an anti-PD-1 antibody.

The term "progression-free survival," which can be abbreviated as PFS, as used herein refers to the length of time during and after the treatment of a solid tumor (*i.e.,* melanoma) that a patient lives with the disease but it does not get worse.

"Dosing interval," as used herein, means the amount of time that elapses between multiple doses of a formulation disclosed herein being administered to a subject. Dosing interval can thus be indicated as ranges.

The term "dosing frequency" as used herein refers to the frequency of administering doses of a formulation disclosed herein in a given time. Dosing frequency can be indicated as the number of doses per a given time, *e.g.,* once a week or once in two weeks, etc.

The terms "about once a week," "once about every week," "once about every two weeks," or any other similar dosing interval terms as used herein means approximate number, and "about once a week" or "once about every week" can include every seven days $\pm$ two days, *i.e.*, every five days to every nine days. The dosing frequency of "once a week" thus can be every five days, every six days, every seven days, every eight days, or every nine days. "Once about every four weeks" can include every 28 days $\pm$ 3 days, *i.e.,* every 25 days to every 31 days. Similar approximations apply, for example, to once about every three weeks, once about every four weeks, once about every five weeks, once about every six weeks and once about every twelve weeks. In some embodiments, a dosing interval of once about every six weeks or once about every twelve weeks means that the first dose can be administered any day in the first week, and then the next dose can be administered any day in the sixth or twelfth week, respectively. In other embodiments, a dosing interval of once about every six weeks or once about every twelve weeks means that the first dose is administered on a particular day of the first week (e.g., Monday) and then the next dose is administered on the same day of the sixth or twelfth weeks (i.e., Monday), respectively.

A "cancer" refers a broad group of various diseases characterized by the uncontrolled growth of abnormal cells in the body. Unregulated cell division and growth results in the formation of malignant tumors that invade neighboring tissues and may also metastasize to distant parts of the body through the lymphatic system or bloodstream. A "cancer" or "cancer tissue" can include a tumor.

The term "tumor" as used herein refers to any mass of tissue that results from excessive cell growth or proliferation, either benign (non-cancerous) or malignant (cancerous), including pre-cancerous lesions.

The term "LAG-3 positive" or "LAG-3 expression positive," relating to LAG-3 expression, refers to the proportion of cells in a test tissue sample comprising tumor cells and tumor-infiltrating inflammatory cells above which the tissue sample is scored as expressing LAG-3. In some embodiments, for LAG-3 expression assayed by immunohistochemistry (IHC), the LAG-3 positive tumor or LAG-3 expression positive tumor means that at least about 0.01%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 40%, at least about 50%, at least about 60%, at least about 70%, at least about 80%, at least about 90%, or 100% of the total number of cells express LAG-3. In other embodiments, for LAG-3 expression assayed by immunohistochemistry (IHC) or flow cytometry, the LAG-3 positive tumor or LAG-3 expression positive tumor means that at least about 0.01%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, at least about 30%, at least about 40%, at least about 50%, at least about 60%, at least about 70%, at least about 80%, at least about 90%, or 100% of the total number of tumor-associated inflammatory cells (e.g., T cells, CD8+ T cells, CD4+ T cells, FOXP3+ cells) express LAG-3. LAG-3 positive tumor or LAG-3 expression positive tumor is also herein as tumor expressing LAG-3. In some embodiments, the LAG-3 positive tumor or LAG-3 expression positive tumor means that at least about 0.1% to at least about 20% of the total number of cells express LAG-3. In some embodiments, a LAG-3 positive tumor or LAG-3 expression positive tumor means that at least about 0.1% to at least about 20% of the total number of tumor-associated inflammatory cells (e.g., T cells, CD8+ T cells, CD4+ T cells, FOXP3+ cells) express LAG-3. In certain embodiments, a LAG-3 positive tumor or LAG-3 expression positive tumor means that at least about 0.1% to at least about 10% of the total number of cells express LAG-3. In certain embodiments, a LAG-3 positive tumor or LAG-3 expression positive tumor means that at least about 0.1% to at least about 10% of the total number of tumor-infiltrating inflammatory cells (e.g., T cells, CD8+ T cells, CD4+ T cells, FOXP3+ cells) express LAG-3. In certain embodiments, a LAG-3 positive tumor or LAG-3 expression positive tumor means that at least about 0.1% to at least about 10% of the total number of cells express LAG-3 by % tumor area. In some embodiments, a LAG-3 positive or LAG-3 expression positive tumor means that at least about 1% of the total number of cells express LAG-3 on the cell surface. In some embodiments, a LAG-3 positive or LAG-3 expression positive tumor means that at least about 1% of the total number of tumor-infiltrating inflammatory cells (e.g., T cells, CD8+ T cells, CD4+ T cells, FOXP3+ cells) express LAG-3 on the cell surface. In some embodiments, a LAG-3 positive or LAG-3 expression positive tumor means that at least about 1% of the total number of cells express LAG-3 by % tumor area. In other embodiments, a LAG-3 positive or LAG-3 expression positive tumor means that at least about 5% of the total number of cells express LAG-3 on the cell surface. In other embodiments, a LAG-3 positive or LAG-3 expression positive tumor means that at least about 5% of the total number of tumor-infiltrating inflammatory cells (e.g., T cells, CD8+ T cells, CD4+ T cells, FOXP3+ cells) express LAG-3 on the cell surface. In some embodiments, a LAG-3 positive or LAG-3 expression positive tumor means that at least about 5% of the total number of cells express LAG-3 by % tumor area. In one particular embodiment, LAG-3 positive or LAG-3 expression positive tumor means that at least about 1%, or in the range of 1-5% of the total number of cells express LAG-3 on the cell surface. In one particular embodiment, LAG-3 positive or LAG-3 expression positive tumor means that at least about 1%, or in the range of 1- 5% of the total number of tumor-infiltrating inflammatory cells (e.g., T cells, CD8+ T cells, CD4+ T cells, FOXP3+ cells) express LAG-3 on the cell surface.

"LAG-3 negative" or "LAG-3 expression negative," refers to the proportion of cells in a test tissue sample comprising tumor cells and tumor- infiltrating inflammatory cells that are not LAG-3 positive or LAG-3 expression positive.

The term "PD-L1 positive" or "PD-L1 expression positive," relating to cell surface PD-L1 expression, refers to the proportion of cells in a test tissue sample comprising tumor cells and tumor- infiltrating inflammatory cells above which the sample is scored as expressing cell surface PD-L1. For cell surface expression assayed by immunohistochemistry (IHC), e.g., with the mAb 28- 8, the PD-L1 positive tumor or PD-L1 expression positive tumor means that at least about 0.01%, at least about 0.05%, at least about 0.1%, at least about 0.5%, at least about 1%, at least about 2%, at least about 3%, at least about 4%, at least about 5%, at least about 6%, at least about 7%, at least about 8%, at least about 9%, at least about 10%, at least about 15%, at least about 20%, at least about 25%, or at least about 30% of the total number of cells express PD-L1. PD-L1 positive tumor or PD-L1 expression positive tumor can also be expressed herein as tumor expressing PD-L1. In other embodiments, the PD-L1 positive tumor or PD-L1 expression positive tumor means that at least about 0.1% to at least about 20% of the total number of cells express PD-L1. In certain embodiments, the PD-L1 positive tumor or PD-L1 expression positive tumor means that at least about 0.1% to at least about 10% of the total number of cells express PD-L1. In some embodiments, the PD-L1 positive or PD-L1 expression positive tumor means that at least about 1% of the total number of cells express PD-L1 on the cell surface. In other embodiments, the PD-L1 positive or PD-L1 expression positive tumor means that at least about 5% of the total number of cells express PD-L1 on the cell surface. In one particular embodiment, PD-L1 positive or PD-L1 expression positive tumor means that at least about 1%, or at least about 1- 5% of the total number of cells express PD-L1 on the cell surface.

An "immune response" refers to the action of a cell of the immune system (for example, T lymphocytes, B lymphocytes, natural killer (NK) cells, macrophages, eosinophils, mast cells, dendritic cells and neutrophils) and soluble macromolecules produced by any of these cells or the liver (including antibodies, cytokines, and complement) that results in selective targeting, binding to, damage to, destruction of, and/or elimination from a vertebrate's body of invading pathogens, cells or tissues infected with pathogens, cancerous or other abnormal cells, or, in cases of autoimmunity or pathological inflammation, normal human cells or tissues.

A "tumor-infiltrating inflammatory cell" or "tumor-associated inflammatory cell" is any type of cell that typically participates in an inflammatory response in a subject and which infiltrates tumor tissue. Such cells include tumor-infiltrating lymphocytes (TILs), macrophages, monocytes, eosinophils, histiocytes and dendritic cells.

The use of the alternative (e.g., "or") should be understood to mean either one, both, or any combination thereof of the alternatives. As used herein, the indefinite articles "a" or "an" should be understood to refer to "one or more" of any recited or enumerated component.

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

The terms "about" or "comprising essentially of" refer to a value or composition that is within an acceptable error range for the particular value or composition as determined by one of ordinary skill in the art, which will depend in part on how the value or composition is measured or determined, *i.e.,* the limitations of the measurement system. For example, "about" or "comprising essentially of" can mean within 1 or more than 1 standard deviation per the practice in the art. Alternatively, "about" or "comprising essentially of" can mean a range of up to 10% or 20% (*i.e.,* ±10% or ±20%). For example, about 3mg can include any number between 2.7 mg and 3.3 mg (for 10%) or between 2.4 mg and 3.6 mg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As described herein, any concentration range, percentage range, ratio range or integer range is to be understood to include the value of any integer within the recited range and, when appropriate, fractions thereof (such as one-tenth and one-hundredth of an integer), unless otherwise indicated.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 5th ed., 2013, Academic Press; and the Oxford Dictionary Of Biochemistry And Molecular Biology, 2006, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. The headings provided herein are not limitations of the various aspects of the disclosure, which can be had by reference to the specification as a whole. Accordingly, the terms defined immediately below are more fully defined by reference to the specification in its entirety.

Various aspects of the invention are described in further detail in the following subsections.

## 2. Methods of the Invention

In one aspect, the present invention is directed to a method for treating metastatic or unresectable melanoma in a subject in need thereof. A combination therapy of a LAG-3 inhibitor (e.g., anti-LAG-3 antibody) and a PD-1 pathway inhibitor (e.g., anti-PD-1 antibody) results in better therapeutic outcomes (e.g., objective response rate and disease control rate) for afflicted patients.

In one embodiment, the invention includes a method of selecting a metastatic or unresectable melanoma in a human patient for immunotherapy, comprising determining the level of LAG-3 and/or PD-L1 expression in a tumor sample.

In one embodiment, the invention includes a method of treating a metastatic or unresectable melanoma in a human patient, comprising: administering to the patient an immunotherapy comprising a LAG-3 inhibitor (an anti-LAG-3 antibody) and a PD-1 pathway inhibitor (an anti-PD-1 antibody).

In one embodiment, the invention includes a method of treating metastatic or unresectable melanoma in a human patient in need thereof, comprising: (a) determining the level of LAG-3 expression or the level of LAG-3 and PD-L1 expression in a tumor sample; and (b) administering to the patient a therapeutically effective amount of a LAG-3 inhibitor and a PD-1 pathway inhibitor.

In one embodiment, the invention includes a method of treating metastatic or unresectable melanoma in a human patient in need thereof, comprising: (a) determining the level of LAG-3 expression or the level of LAG-3 and PD-L1 expression in a tumor sample; (b) determining the presence of tumor cells expressing the BRAF V600 mutation; and (c) administering to the patient a therapeutically effective amount of a LAG-3 inhibitor and a PD-1 pathway inhibitor.

In certain embodiments, the invention includes a method for extending a progression-free survival period by over 12 months in a human patient afflicted with a metastatic or unresectable melanoma comprising administering to the patient an immunotherapy disclosed herein, wherein the patient demonstrates progression-free survival for over 12 months. In some embodiments, the progression-free survival of the patient can be extended, after the administration, for over about 13 months, about 14 months, about 15 months, about 16 months, about 17 months, about 18 months, about 2 years, about 3 years, about 4 years, about 5 years, about 6 years, about 7 years, about 8 years, about 9 years, or about 10 years as compared to standard of care therapy.

In still other embodiments, the invention is includes a method for reducing a tumor size at least by 10% in a human patient afflicted with metastatic or unresectable melanoma comprising administering to the patient an immunotherapy disclosed herein, wherein the administration reduces the tumor size at least about 10%, about 20%, about 30%, about 40%, about 50%, about 60%, about 70%, about 80%, about 90%, or 100% compared to the tumor size prior to the administration.

In some embodiments, the method comprises identifying the patient as having tumor infiltrating lymphocytes or tumor cells that express or contain a particular marker. For example, in some embodiments, the melanoma is LAG-3 positive. In some embodiments, the melanoma is PD-L1 positive. In some embodiments, the melanoma is LAG-3 positive PD-L1 positive. In some embodiments, the melanoma contains a BRAF V600 mutation. In some embodiments, the melanoma is LAG-3 positive and expresses the BRAF V600 mutation. In some embodiments, the melanoma is LAG-3 positive and contains tumor cells that express wild-type BRAF. In some embodiments, the melanoma is LAG-3 positive, PD-L1 positive and contains wild-type BRAF. In some embodiments, the melanoma is LAG-3 positive, PD-L1 positive and contains a BRAF V600 mutation. In some embodiments, the melanoma is PD-L1 positive and contains a wild-type BRAF. In some embodiments, the melanoma is PD-L1 positive and contains a BRAF V600 mutation. In some embodiments, the LAG-3 inhibitor is an anti-LAG-3 antibody and the PD-1 pathway inhibitor is an anti-PD-1 antibody.

The invention can also include a method of preventing a relapse and/or inducing a remission in a patient comprising administering to the patient an immunotherapy disclosed herein.

In certain embodiments, the invention includes a method for increasing an objective response rate to be higher than 55% in a patient population, wherein each patient of the patient population is afflicted with metastatic or unresectable melanoma, in a cancer treatment comprising administering to the patient an immunotherapy disclosed herein, wherein each patient is identified as having metastatic or unresectable melanoma and wherein the objective response rate is higher than about 55%, about 60%, about 65%, about 70%, or about 75% or higher. In some embodiments, the method comprises identifying the patient as having a LAG-3 positive, PD-L1 positive tumor prior to the administration. In some embodiments, the tumor expresses the BRAF V600 mutation. In some embodiments, the immunotherapy comprises administering a therapeutically effective amount of a LAG-3 inhibitor and a PD-1 pathway inhibitor. In some embodiments, the LAG-3 inhibitor is an anti-LAG-3 antibody and the PD-1 pathway inhibitor is an anti-PD-1 antibody.

In certain embodiments, the invention includes a method for increasing disease control rate to be higher than 55% in a patient population, wherein each patient of the patient population is afflicted with metastatic or unresectable melanoma, in a cancer treatment comprising administering to the patient an immunotherapy disclosed herein, wherein each patient is identified as having metastatic or unresectable melanoma and wherein the disease control rate is higher than about 55%, about 60%, about 65%, about 70%, or about 75% or higher. In some embodiments, the method comprises identifying the patient as having a LAG-3 positive PD-L1 positive tumor prior to the administration. In some embodiments, the tumor contains the BRAF V600 mutation. In some embodiments, the immunotherapy comprises administering a therapeutically effective amount of a LAG-3 inhibitor and a PD-1 pathway inhibitor. In some embodiments, the LAG-3 inhibitor is an anti-LAG-3 antibody and the PD-1 pathway inhibitor is an anti-PD-1 antibody.

In other embodiments, each patient in the methods experiences (i) extended progression-free survival for over 12 months, (ii) tumor size reduction at least about 10%, about 20%, about 30%, about 40%, or about 50% or higher compared to the tumor size prior to the administration, or (iii) both.

The methods of the invention, as a result of the administration of an immunotherapy disclosed herein, can treat the metastatic or unresectable melanoma tumor, reduce the tumor size, inhibit growth of the tumor, eliminate the tumor from the patient, prevent a relapse of a tumor, induce a remission in a patient, or any combination thereof. In certain embodiments, the administration of an immunotherapy disclosed herein induces a complete response. In other embodiments, the administration of the immunotherapy disclosed herein induces a partial response. In some embodiments, the immunotherapy comprises administering a therapeutically effective amount of a LAG-3 inhibitor and a PD-1 pathway inhibitor. In some embodiments, the LAG-3 inhibitor is an anti-LAG-3 antibody and the PD-1 pathway inhibitor is an anti-PD-1 antibody.

In some embodiments, the LAG-3 and/or PD-L1 positive tumor comprises at least about 1%, at least about 2%, at least about 3%, at least about 4%, at least about 5%, at least about 7%, at least about 10%, at least about 15%, at least about 20%, at least about 25%, at least about 30%, at least about 40%, at least about 50%, at least about 60%, at least about 70%, at least about 80%, at least about 90%, or 100% cells expressing LAG-3. In some embodiments, the cells expressing LAG-3 are tumor infiltrating lymphocytes. In some embodiments, the cells expressing PD-L1 are tumor cells.

In some embodiments, LAG-3 and/or PD-L1 expression is determined by receiving the results of an assay capable of determining LAG-3 and/or PD-L1 expression.

Method for determining PD-L1 expression in a tumor sample, methods for identifying the patient as having a PD-L1 positive malignant tumor, and methods for determining PD-L1 expression in a malignant tumor have been disclosed in PCT/US2016/029878, the teachings of which are hereby incorporated by reference.

In order to assess expression of LAG-3 and PD-L1, and/or whether the cancer contains a BRAF V600 mutation, in one embodiment, a test tissue sample is obtained from the patient who is in need of the therapy. In some embodiments, a test tissue sample includes, but is not limited to, any clinically relevant tissue sample, such as a tumor biopsy, a core biopsy tissue sample, a fine needle aspirate, or a sample of bodily fluid, such as blood, plasma, serum, lymph, ascites fluid, cystic fluid, or urine. In some embodiments, the test tissue sample is from a primary tumor. In some embodiments, the test tissue sample is from a metastasis. In some embodiments, test tissue samples are taken from a subject at multiple time points, for example, before treatment, during treatment, and/or after treatment. In some embodiments, test tissue samples are taken from different locations in the subject, for example, a sample from a primary tumor and a sample from a metastasis in a distant location.

In some embodiments, the test tissue sample is a paraffin-embedded fixed tissue sample. In some embodiments, the test tissue sample is a formalin-fixed paraffin embedded (FFPE) tissue sample. In some embodiments, the test tissue sample is a fresh tissue (e.g., tumor) sample. In some embodiments, the test tissue sample is a frozen tissue sample. In some embodiments, the test tissue sample is a fresh frozen (FF) tissue (e.g., tumor) sample. In some embodiments, the test tissue sample is a cell isolated from a fluid. In some embodiments, the test tissue sample comprises circulating tumor cells (CTCs). In some embodiments, the test tissue sample comprises tumor-infiltrating lymphocytes (TILs). In some embodiments, the test tissue sample comprises tumor cells and tumor-infiltrating lymphocytes (TILs). In some embodiments, the test tissue sample comprises circulating lymphocytes. In some embodiments, the test tissue sample is an archival tissue sample. In some embodiments, the test tissue sample is an archival tissue sample with known diagnosis, treatment, and/or outcome history. In some embodiments, the sample is a block of tissue. In some embodiments, the test tissue sample is dispersed cells. In some embodiments, the sample size is from about 1 cell to about $1 \times 10^6$ cells or more. In some embodiments, the sample size is about 1 cell to about $1 \times 10^5$ cells. In some embodiments, the sample size is about 1 cell to about 10,000 cells. In some embodiments, the sample size is about 1 cell to about 1,000 cells. In some embodiments, the sample size is about 1 cells to about 100 cells. In some embodiments, the sample size is about 1 cell to about 10 cells. In some embodiments, the sample size is a single cell.

In another embodiment, the assessment of LAG-3, PD-L1 and/or BRAF V600 status can be achieved without obtaining a test tissue sample. In some embodiments, selecting a suitable patient includes (i) optionally providing a test tissue sample obtained from a patient with cancer of the tissue, the test tissue sample comprising tumor cells and/or tumor-infiltrating inflammatory cells; and (ii) assessing the proportion of cells in the test tissue sample that express LAG-3, PD-L1 and/or BRAF V600 based on an assessment that the proportion of cells in the test tissue sample is higher than a predetermined threshold level.

In any of the methods comprising the measurement of LAG-3, PD-L1 and/or BRAF V600 status in a test tissue sample, however, it should be understood that the step comprising the provision of a test tissue sample obtained from a patient is an optional step. That is, in certain embodiments the method includes this step, and in other embodiments, this step is not included in the method. It should also be understood that in certain embodiments the "measuring" or "assessing" step to identify, or determine the number or proportion of, cells in the test tissue sample that express LAG-3 and/or PD-L1 is performed by a transformative method of assaying for LAG-3 and/or PD-L1, for example by performing a reverse transcriptase-polymerase chain reaction (RT-PCR) assay or an IHC assay. In certain other embodiments, no transformative step is involved and LAG-3 and/or PD-L1 expression is assessed by, for example, reviewing a report of test results from a laboratory. In some embodiments, LAG-3 and/or PD-L1 expression is assessed by reviewing the results of an immunohistochemistry assay from a laboratory. In certain embodiments, the steps of the methods up to, and including, assessing LAG-3 and/or PD-L1 expression provides an intermediate result that may be provided to a physician or other healthcare provider for use in selecting a suitable candidate for the combination therapy of a LAG-3 inhibitor and a PD-1 pathway inhibitor. In certain embodiments, the steps that provide the intermediate result is performed by a medical practitioner or someone acting under the direction of a medical practitioner. In other embodiments, these steps are performed by an independent laboratory or by an independent person such as a laboratory technician. In some embodiments, the presence of a BRAF V600 mutation is performed using parallel approaches for LAG-3 and/or PD-L1.

In certain embodiments of any of the present methods, the proportion of cells that express LAG-3 and PD-L1, and/or contain a BRAF V600 mutation is assessed by performing an assay to detect the presence of LAG-3, PD-L1 and/or BRAF RNA. In further embodiments, the presence of LAG-3, PD-L1 and/or BRAF RNA is detected by RT-PCR, *in situ* hybridization or RNase protection. In some embodiments, the presence of LAG-3, PD-L1 and/or BRAF RNA is detected by an RT-PCR based assay. In some embodiments, scoring the RT-PCR based assay comprises assessing the level of LAG-3, PD-L1 and/or BRAF RNA expression in the test tissue sample relative to a predetermined level. In some embodiments, expression of one or more of LAG-3, PD-L1 and BRAF V600 is assessed using gene expression profiling.

In other embodiments, the proportion of cells that express LAG-3 and PD-L1, and/or contain a BRAF V600 mutation is assessed by performing an assay to detect the presence of LAG-3 and PD-L1, and/or contain a BRAF V600 mutation polypeptide. In further embodiments, the presence of LAG-3, PD-L1 and/or BRAF V600 polypeptide is detected by IHC, enzyme-linked immunosorbent assay (ELISA), *in vivo* imaging, or flow cytometry. In some embodiments, LAG-3, PD-L1 expression and BRAF V600 status is assayed by IHC. In other embodiments of all of these methods, cell surface expression of LAG-3 and PD-L1 and/or the presence of a BRAF V600 mutation is assayed using, e.g., IHC or *in vivo* imaging.

In other embodiments, the proportion of cells that express LAG-3 and PD-L1 and/or contain a BRAF V600 mutation in the test tissue sample is assessed by flow cytometry. In some embodiments, the test tissue sample assayed by flow cytometry comprises tumor infiltrating immune cells. In some embodiments, the malignant tumor is a hematological malignancy and the tissue sample assayed by flow cytometry comprises peripheral blood cells. In some embodiments, the flow cytometry is a multiplex assay. In some embodiments, scoring the flow cytometry comprises detecting the expression of markers comprising LAG-3, CD4, CD8, FOXP3, and any combination thereof. In some embodiments, scoring the flow cytometry comprises assessing the proportion of T cells in the test tissue sample that express LAG-3 and PD-L1, and/or contain a BRAF V600 mutation. In some embodiments, scoring the flow cytometry comprises assessing the proportion of CD8+ T cells in the test tissue sample that express LAG-3 and PD-L1, and/or contain a BRAF V600 mutation. In some embodiments, scoring the flow cytometry comprises assessing the proportion of CD4+ T cells in the test tissue sample that express LAG-3 and PD-L1, and/or contain a BRAF V600 mutation. In some embodiments, scoring the flow cytometry comprises assessing the proportion of FOXP3+ T cells in the test tissue sample that express LAG-3 and PD-L1, and/or contain a BRAF V600 mutation.

In certain embodiments of any of the present methods, the proportion of cells that express LAG-3, PD-L1 and/or contain a BRAF V600 in the test tissue sample is assessed by performing an assay to detect the presence of LAG-3 polypeptide. In some embodiments, the presence of LAG-3, PD-L1 and/or BRAF V600 polypeptide is detected by an immunohistochemistry assay. In some embodiments, the test tissue sample is a tumor biopsy. In some embodiments, the test tissue sample is a formalin-fixed paraffin embedded (FFPE) sample.

In some embodiments, the immunohistochemistry assay is a monoplex assay. In some embodiments, the immunohistochemistry assay is a multiplex assay. In some embodiments, the multiplex immunohistochemistry assay is capable of detecting the presence of CD4, CD8, FOXP3, or any combination thereof.

In some embodiments, the immunohistochemistry assay comprises contacting the tumor sample with the 17B4 mouse anti-human LAG-3 IgG1 monoclonal antibody. In some embodiments, the immunohistochemistry assay comprises contacting the tumor sample with an anti-LAG-3 antibody comprising heavy and light chain variable regions comprising the sequences set forth in SEQ ID NOs: 3 and 5, respectively. In some embodiments, the immunohistochemistry assay comprises contacting the tumor sample with the SP346 rabbit anti-human LAG-3 IgG monoclonal antibody. In some embodiments, the immunohistochemistry assay comprises contacting the tumor sample with the 11E3 (Novusbio), 874501 (Novusbio), or EPR4392(2) (Abcam) anti-human LAG-3 monoclonal antibody. In some embodiments, the immunohistochemistry assay comprises contacting the tumor sample with reagents in the Dako PD-L1 IHC 28-8 kit to assay for PD-L1 expression.

In some embodiments, the immunohistochemistry assay is scored at a low magnification. In some embodiments, low magnification is about 20X. In some embodiments, the immunohistochemistry assay is scored at high magnification. In some embodiments, high magnification is about 40X.

In some embodiments, the immunohistochemistry assay is scored by an image analysis software. In some embodiments, the immunohistochemistry assay is scored by pathologist visual immune score. In some embodiments, the immunohistochemistry assay is scored manually.

In some embodiments, scoring the immunohistochemistry assay comprises assessing the proportion of cells in the test tissue sample that express LAG-3 and PD-L1, and/or contain a BRAF V600 mutation. In some embodiments, scoring the immunohistochemistry assay comprises assessing the proportion of immune cells in the test tissue sample that express LAG-3 and PD-L1, and/or contain a BRAF V600 mutation. In some embodiments, scoring the immunohistochemistry assay comprises assessing the proportion of T cells in the test tissue sample that express LAG-3 and PD-L1, and/or contain a BRAF V600 mutation. In some embodiments, scoring the immunohistochemistry assay comprises assessing the proportion of CD8+ T cells in the test tissue sample that express LAG-3. In some embodiments, scoring the immunohistochemistry assay comprises assessing the proportion of CD4+ T cells in the test tissue sample that express LAG-3. In some embodiments, scoring the immunohistochemistry assay comprises assessing the proportion of FOXP3+ T cells in the test tissue sample that express LAG-3.

In some embodiments, the immunohistochemistry assay is a multiplex assay that further comprises detecting the expression of MHC Class II by the tumor cells. In some embodiments, scoring the immunohistochemistry assay comprises assessing the proportion of cells in the test tissue sample that expresses MHC Class II. In some embodiments, scoring the immunohistochemistry assay comprises assessing the proportion of non-immune cells in the test tissue sample that expresses MHC Class II.

In a particular embodiments, the expression of fibrinogen-like protein 1 (FGL1) by the tumor cells is measured.

Imaging techniques have provided important tools in cancer research and treatment. Recent developments in molecular imaging systems, including positron emission tomography (PET), single-photon emission computed tomography (SPECT), fluorescence reflectance imaging (FRI), fluorescence-mediated tomography (FMT), bioluminescence imaging (BLI), laser-scanning confocal microscopy (LSCM) and multiphoton microscopy (MPM), will likely herald even greater use of these techniques in cancer research. Some of these molecular imaging systems allow clinicians to not only see where a tumor is located in the body, but also to visualize the expression and activity of specific molecules, cells, and biological processes that influence tumor behavior and/or responsiveness to therapeutic drugs (Condeelis and Weissleder, Cold Spring Harb. Perspect. Biol. 2(12):a003848 (2010)). Antibody specificity, coupled with the sensitivity and resolution of PET, makes immunoPET imaging particularly attractive for monitoring and assaying expression of antigens in tissue samples (McCabe and Wu, Cancer Biother. Radiopharm. 25(3):253-61 (2010); Olafsen et al., Protein Eng. Des. Sel. 23(4):243-9 (2010)). In certain embodiments of any of the present methods, LAG-3, PD-L1 and/or BRAF V600 expression is assayed by immunoPET imaging. In certain embodiments of any of the present methods, the proportion of cells in a test tissue sample that express LAG-3, PD-L1 and/or BRAF V600 is assessed by performing an assay to determine the presence of LAG-3, PD-L1 and/or BRAF V600 polypeptide on the surface of cells in the test tissue sample. In certain embodiments, the test tissue sample is a FFPE tissue sample. In other embodiments, the presence of LAG-3, PD-L1 and/or BRAF V600 polypeptide is determined by IHC assay. In further embodiments, the IHC assay is performed using an automated process.

**3.** Assaying LAG-3 and PD-L1 expression and/or the presence of a BRAF V600 mutation by Automated IHC

In one embodiment of the present methods, an automated IHC method is used to assay the expression of LAG-3 and PD-L1, and/or the presence a BRAF V600 mutation in FFPE tissue specimens. This disclosure provides methods for detecting the presence of human LAG-3, PD-L1 and/or BRAF V600 in a test tissue sample, or quantifying the level of human LAG-3, PD-L1 and/or BRAF V600 antigen or the proportion of cells in the sample that express the antigen, which methods comprise contacting the test sample, and a negative control sample, with a mAb that specifically binds to human LAG-3, PD-L1 and/or BRAF V600, under conditions that allow for formation of a complex between the antibody or portion thereof and human LAG-3, PD-L1 and/or BRAF V600. In certain embodiments, the test and control tissue samples are FFPE samples. The formation of a complex is then detected, wherein a difference in complex formation between the test sample and the negative control sample is indicative of the presence of human LAG-3, PD-L1 and/or BRAF V600 antigen in the sample. Various methods are used to quantify LAG-3, PD-L1 and/or BRAF V600.

In a particular embodiment, the automated IHC method comprises: (a) deparaffinizing and rehydrating mounted tissue sections in an autostainer; (b) retrieving antigen in an autostainer; (c) setting up reagents on an autostainer; and (d) running the autostainer to include steps of neutralizing endogenous peroxidase in the tissue specimen; blocking non-specific protein-binding sites on the slides; incubating the slides with primary Ab; incubating with a postprimary blocking agent; incubating with a postprimary antibody detection agent, such as another antibody that may or may not be conjugated to a detection enzyme; incubating with a polymeric-enzyme detection reagent; adding a chromogen substrate and developing; and counterstaining with hematoxylin. In some embodiments, the retrieving antigen comprises using any heat based antigen retrieval device.

In some embodiments, for assessing the presence of LAG-3, PD-L1 and/or BRAF V600 in tumor tissue samples, a pathologist examines the number of LAG-3+ tumor infiltrating lymphocytes, PD-L1+ tumor cells and/or BRAF V600+ tumor cells in each field under a microscope and mentally estimates the percentage of cells that are positive, then averages them to come to the final percentage. The different staining intensities are defined as 0/negative, 1+/weak, 2+/moderate, and 3+/strong. Typically, percentage values are first assigned to the 0 and 3+ buckets, and then the intermediate 1+ and 2+ intensities are considered. For highly heterogeneous tissues, the specimen is divided into zones, and each zone is scored separately and then combined into a single set of percentage values. The percentages of negative and positive cells for the different staining intensities are determined from each area and a median value is given to each zone. A final percentage value is given to the tissue for each staining intensity category: negative, 1+, 2+, and 3+. The sum of all staining intensities needs to be 100%.

In some embodiments, staining is also assessed in tumor-infiltrating inflammatory cells such as macrophages and lymphocytes. Macrophages and lymphocytes are assessed for LAG-3, PD-L1 and/or BRAF V600 staining and only recorded for all samples as being positive or negative for each cell category. Staining is also characterized according to an outside/inside tumor immune cell designation. "Inside" means the immune cell is within the tumor tissue and/or on the boundaries of the tumor region without being physically intercalated among the tumor cells. "Outside" means that there is no physical association with the tumor, the immune cells being found in the periphery associated with connective or any associated adjacent tissue.

In certain embodiments of these scoring methods, the samples are scored by two or more pathologists operating independently, and the scores are subsequently consolidated. In certain other embodiments, the identification of positive and negative cells is scored using appropriate software.

A histoscore (H-score) is used as a more quantitative measure of the IHC data. The histoscore is calculated as follows:

$$\text{Histoscore} = [(\% \text{ tumor x 1 (low intensity)}) + (\% \text{ tumor x 2 (medium intensity)})$$
$$+ (\% \text{ tumor x 3 (high intensity)})]$$

To determine the histoscore, the pathologist estimates the percentage of stained cells in each intensity category within a specimen. Because expression of most biomarkers is heterogeneous the histoscore is a truer representation of the overall expression. The final histoscore range is 0 (minimum score, no expression) to 300 (maximum score, strong and inclusive expression).

**4.** LAG-3 inhibitors

In one aspect, the invention features methods of using a LAG-3 inhibitor in the treatment of malignant tumors. As used herein LAG-3 inhibitor includes, but is not limited to, LAG-3 binding agents and soluble LAG-3 polypeptides. LAG-3 binding agents include antibodies that specifically bind to LAG-3.

In some embodiments, a LAG-3 inhibitor is a LAG-3-binding agent, for example an anti-LAG-3 antibody. In some embodiments, the LAG-3 inhibitor is a soluble LAG-3 polypeptide, for example, a LAG-3-Fc fusion polypeptide capable of binding to MHC Class II.

Anti-human-LAG-3 antibodies (or VH/VL domains derived therefrom) suitable for use in the invention can be generated using methods well known in the art. Alternatively, art recognized anti-LAG-3 antibodies can be used.

In some embodiments, the anti-LAG-3 antibody is BMS-986016 comprising heavy and light chains comprising the sequences shown in SEQ ID NOs:1 and 2, respectively, or variants thereof, as described in PCT/US13/48999, the teachings of which are hereby incorporated by reference. In some embodiments, the BMS-986016 antibody does not comprise the heavy chain terminal lysine amino acid of SEQ ID NO:1.

In other embodiments, the antibody has the heavy and light chain CDRs or variable regions of BMS-986016 (relatlimab). Accordingly, in one embodiment, the antibody comprises CDR1, CDR2, and CDR3 domains of the VH region of BMS-986016 having the sequence set forth in SEQ ID NO:3, and CDR1, CDR2 and CDR3 domains of the VL region of BMS-986016 having the sequence set forth in SEQ ID NO:5. In another embodiment, the antibody comprises CDR1, CDR2 and CDR3 domains comprising the sequences set forth in SEQ ID NOs:7, 8, and 9, respectively, and CDR1, CDR2 and CDR3 domains comprising the sequences set forth in SEQ ID NOs:10, 11, and 12, respectively. In another embodiment, the antibody comprises VH and/or VL regions comprising the amino acid sequences set forth in SEQ ID NO:3 and/or SEQ ID NO: 5, respectively. In another embodiment, the antibody comprises heavy chain variable (VH) and/or light chain variable (VL) regions encoded by the nucleic acid sequences set forth in SEQ ID NO:4 and/or SEQ ID NO:6, respectively. In another embodiment, the antibody competes for binding with and/or binds to the same epitope on LAG-3 as the above-mentioned antibodies. In another embodiment, the antibody binds an epitope of human LAG-3 comprising the amino acid sequence PGHPLAPG (SEQ ID NO:27). In another embodiment, the antibody binds an epitope of human LAG-3 comprising the amino acid sequence HPAAPSSW (SEQ ID NO:28) or PAAPSSWG (SEQ ID NO:29). In an embodiment, the antibody comprises (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; and (e) a light chain variable region CDR2 comprising the sequence set forth in SEQ ID NO:11.

In another embodiment, the antibody has at least about 90% variable region amino acid sequence identity with the above-mentioned antibodies (e.g., at least about 90%, 95% or 99% variable region identity with SEQ ID NO:3 or SEQ ID NO:5).

In some embodiments, the anti-LAG-3 antibody cross-competes with BMS-986016 (relatlimab) for binding to human LAG-3. In other embodiments, the anti- LAG-3 antibody binds to the same epitope as BMS-986016 (relatlimab). In some embodiments, the anti- LAG-3 antibody is a chimeric antibody, a humanized antibody, or a human monoclonal antibody. In other embodiments, the anti- LAG-3 comprises a heavy chain constant region of a human IgG1 isotype or a human IgG4 isotype. In particular embodiments, the anti-LAG-3 antibody thereof is BMS-986016 (relatlimab). In certain embodiments, the anti- LAG-3 antibody is a biosimilar. In some embodiments, the anti-LAG-3 antibody is a biosimilar of BMS-986016 (relatlimab).

In some embodiments, art recognized anti-LAG-3 antibodies can be used in the therapeutic methods of the invention. For example, the anti-human LAG-3 antibody described in US2011/0150892 A1, and referred to as monoclonal antibody 25F7 (also known as "25F7" and "LAG-3.1) can be used. Other art recognized anti-LAG-3 antibodies that can be used include IMP731 (H5L7BW) described in US 2011/007023, MK-4280 (28G-10) described in WO2016028672, aLAG3(0414) and aLAG3(0416) described in WO2018185046, anti-PD1/LAG3 0927 described in WO2018185043, REGN3767 described in Journal for ImmunoTherapy of Cancer, (2016) Vol. 4, Supp. Supplement 1 Abstract Number: P195, BAP050 described in WO2017/019894, IMP-701 (LAG-525), aLAG3(0414), aLAG3(0416), Sym022, TSR-033, TSR-075, XmAb22841, MGD013, BI754111, FS118, P 13B02-30, AVA-017 and GSK2831781. These and other anti-LAG-3 antibodies useful in the claimed invention can be found in, for example: WO2016/028672, WO2017/106129, WO2017/062888, WO2009/044273, WO2018/069500, WO2016/126858, WO2014/179664, WO2016/200782, WO2015/200119, WO2017/019846, WO2017/198741, WO2017/220555, WO2017/220569, WO2018/071500, WO2017/015560, WO2017/025498, WO2017/087589, WO2017/087901, WO2018/083087, WO2017/149143, WO2017/219995, US2017/0260271, WO2017/086367, WO2017/086419, WO2018/034227, WO2018185046, WO2018185043, and WO2014/140180. In one embodiment, the LAG-3 antagonist is IMP321 (eftilagimod alpha). The contents of each of these references are incorporated by reference herein in their entirety. In some embodiments of the present invention, the anti-LAG-3 antibody comprises a serine to proline mutation at amino acid residue 228.

Antibodies that compete with any of the above-referenced antibodies for binding to LAG-3 also can be used.

In certain embodiments, an anti-LAG-3 antibody is used to determine LAG-3 expression. In some embodiments, an anti-LAG-3 antibody is selected for its ability to bind to LAG-3 in formalin-fixed, paraffin-embedded (FFPE) tissue specimens. In other embodiments, an anti-LAG-3 antibody is capable of binding to LAG-3 in frozen tissues. In further embodiments, an anti-LAG-3 antibody is capable of distinguishing membrane bound, cytoplasmic, and/or soluble forms of LAG-3.

In some embodiments, an anti-LAG-3 antibody useful for assaying, detecting, and/or quantifying LAG-3 expression in accordance with the methods described herein is the 17B4 mouse IgG1 anti-human LAG-3 monoclonal antibody. See, e.g., J. Matsuzaki, et al.; PNAS 107, 7875 (2010).

## 5. PD-1 pathway inhibitors

In one aspect, the invention features methods of using a PD-1 inhibitor in the treatment of malignant tumors. As used herein "PD-1 pathway inhibitor" includes, but is not limited to, PD-1 binding agents, PD-L1 binding agent and PD-L2 binding agents. PD-1 binding agents include antibodies that specifically bind to PD-1. PD-L1 and PD-L2 binding agents include antibodies that specifically bind to PD-L1 and/or PD-L2, as well as soluble PD-1 polypeptides that bind to PD-L1 and/or PD-L2.

In some embodiments, PD-1 pathway inhibitor is a PD-1-binding agent, for example an anti-PD-1 antibody. In some embodiments, the PD-1 pathway inhibitor is a PD-L1-binding agent, for example, an anti-PD-L1 antibody. In some embodiments, the PD-1 pathway inhibitor is a PD-L2-binding agent, for example an anti-PD-L2 antibody. In further embodiments, the PD-L1-binding agent is a soluble PD-1 polypeptide, for example, a PD-1-Fc fusion polypeptide capable of binding to PD-L1. In further embodiments, the PD-L2-binding agent is a soluble PD-1 polypeptide, for example, a PD-1-Fc fusion polypeptide capable of binding to PD-L2.

Anti-human-PD-1 antibodies (or VH and/or VL domains derived therefrom) suitable for use in the invention can be generated using methods well known in the art. Alternatively, art recognized anti-PD-1 antibodies can be used. For example, monoclonal antibodies 5C4 (referred to herein as Nivolumab or BMS-936558), 17D8, 2D3, 4H1, 4A11, 7D3, and 5F4, described in WO 2006/121168 can be used. Other known PD-1 antibodies include lambrolizumab (MK-3475) described in WO 2008/156712, and AMP-514 described in WO 2012/145493. Further known PD-1 antibodies and other PD-1 inhibitors include those described in, for example, WO 2009/014708, WO 03/099196, WO 2009/114335 and WO 2011/161699, which are herein incorporated by reference.. In one embodiment, the anti-PD-1 antibody is REGN2810. In one embodiment, the anti-PD-1 antibody is PDR001. Another known anti-PD-1 antibody is pidilizumab (CT-011).

In one embodiment, the anti-PD-1 antibody is nivolumab. Nivolumab (also known as "OPDIVO®"; formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., Cancer Immunol Res. 2(9):846-56 (2014)). In another embodiment, the anti-PD-1 antibody or fragment thereof cross-competes with nivolumab. In other embodiments, the anti-PD-1 antibody or fragment thereof binds to the same epitope as nivolumab. In certain embodiments, the anti-PD-1 antibody has the same CDRs as nivolumab.

In some embodiments, the anti-PD-1 antibody comprises heavy and light chains comprising the sequences shown in SEQ ID NOs:17 and 18, respectively, or variants thereof. In some embodiments, the anti-PD-1 antibody comprises (a) a heavy chain variable region CDR1 comprising the sequence set forth in SEQ ID NO:19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24.

In other embodiments, the antibody has heavy and light chain CDRs or variable regions of nivolumab. Accordingly, in one embodiment, the antibody comprises CDR1, CDR2, and CDR3 domains of the VH of nivolumab having the sequence set forth in SEQ ID NO:19, and CDR1, CDR2 and CDR3 domains of the VL of nivolumab having the sequence set forth in SEQ ID NO:21. In another embodiment, the antibody comprises CDR1, CDR2 and CDR3 domains comprising the sequences set forth in SEQ ID NOs:23, 24, and 25, respectively, and CDR1, CDR2 and CDR3 domains comprising the sequences set forth in SEQ ID NOs:26, 27, and 28, respectively. In another embodiment, the antibody comprises VH and/or VL regions comprising the amino acid sequences set forth in SEQ ID NO: 19 and/or SEQ ID NO: 21, respectively. In another embodiment, the antibody comprises heavy chain variable (VH) and/or light chain variable (VL) regions encoded by the nucleic acid sequences set forth in SEQ ID NO:20 and/or SEQ ID NO:22, respectively. In another embodiment, the antibody competes for binding with and/or binds to the same epitope on PD-1 as the above-mentioned antibodies. In another embodiment, the antibody has at least about 90% variable region amino acid sequence identity with the above-mentioned antibodies (e.g., at least about 90%, 95% or 99% variable region identity with SEQ ID NO:19 or SEQ ID NO:21).

In some embodiments, the anti-PD-1 antibody cross-competes with nivolumab for binding to human PD-1. In other embodiments, the anti-PD-1 antibody binds to the same epitope as nivolumab. In some embodiments, the anti-PD-1 antibody is a chimeric antibody, a humanized antibody, or a human monoclonal antibody. In other embodiments, the anti-PD-1 antibody comprises a heavy chain constant region of a human IgG1 isotype or a human IgG4 isotype. In particular embodiments, the anti-PD-1 antibody is nivolumab or pembrolizumab. In some embodiments, the anti-PD-1 antibody is a biosimilar of nivolumab. In some embodiments, the anti-PD-1 antibody is a biosimilar of pembrolizumab.

Anti-PD-1 antibodies that are known in the art can be used in the presently described compositions and methods. Various human monoclonal antibodies that bind specifically to PD-1 with high affinity have been disclosed in U.S. Patent No. 8,008,449. Anti-PD-1 human antibodies disclosed in U.S. Patent No. 8,008,449 have been demonstrated to exhibit one or more of the following characteristics: (a) bind to human PD-1 with a $K_D$ of $1 \times 10^{-7}$ M or less, as determined by surface plasmon resonance using a Biacore biosensor system; (b) do not substantially bind to human CD28, CTLA-4 or ICOS; (c) increase T-cell proliferation in a Mixed Lymphocyte Reaction (MLR) assay; (d) increase interferon-$\gamma$ production in an MLR assay; (e) increase IL-2 secretion in an MLR assay; (f) bind to human PD-1 and cynomolgus monkey PD-1; (g) inhibit the binding of PD-L1 and/or PD-L2 to PD-1; (h) stimulate antigen-specific memory responses; (i) stimulate antibody responses; and (j) inhibit tumor cell growth *in vivo.* Anti-PD-1 antibodies usable in the present disclosure include monoclonal antibodies that bind specifically to human PD-1 and exhibit at least one, in some embodiments, at least five, of the preceding characteristics.

Other anti-PD-1 monoclonal antibodies have been described in, for example, U.S. Patent Nos. 6,808,710, 7,488,802, 8,168,757 and 8,354,509, US Publication No. 2016/0272708, and PCT Publication Nos. WO 2012/145493, WO 2008/156712, WO 2015/112900, WO 2012/145493, WO 2015/112800, WO 2014/206107, WO 2015/35606, WO 2015/085847, WO 2014/179664, WO 2017/020291, WO 2017/020858, WO 2016/197367, WO 2017/024515, WO 2017/025051, WO 2017/123557, WO 2016/106159, WO 2014/194302, WO 2017/040790, WO 2017/133540, WO 2017/132827, WO 2017/024465, WO 2017/025016, WO 2017/106061, WO 2017/19846, WO 2017/024465, WO 2017/025016, WO 2017/132825, and WO 2017/133540 each of which is incorporated by reference in its entirety.

In some embodiments, the anti-PD-1 antibody is selected from the group consisting of nivolumab (also known as OPDIVO®, 5C4, BMS-936558, MDX-1106, and ONO-4538), pembrolizumab (Merck; also known as KEYTRUDA®, lambrolizumab, and MK-3475; *see* WO2008/156712), PDR001 (Novartis; also known as spartalizumab; *see* WO 2015/112900), MEDI-0680 (AstraZeneca; also known as AMP-514; *see* WO 2012/145493), cemiplimab (Regeneron; also known as REGN-2810; *see* WO 2015/112800), JS001 (TAIZHOU JUNSHI PHARMA; *see* Si-Yang Liu et al., J. Hematol. Oncol. 10:136 (2017)), BGB-A317 ("Tislelizumab;" Beigene; *see* WO 2015/35606 and US 2015/0079109), INCSHR1210 (Jiangsu Hengrui Medicine; also known as SHR-1210; *see* WO 2015/085847; Si-Yang Liu et al., J. Hematol. Oncol. 10:136 (2017)), TSR-042 (Tesaro Biopharmaceutical; also known as ANB011; *see* WO2014/179664), GLS-010 (Wuxi/Harbin Gloria Pharmaceuticals; also known as WBP3055; *see* Si-Yang Liu et al., J. Hematol. Oncol. 10:136 (2017)), AM-0001 (Armo), STI-1110 (Sorrento Therapeutics; *see* WO 2014/194302), AGEN2034 (Agenus; *see* WO 2017/040790), MGA012 (Macrogenics, *see* WO 2017/19846), IBI308 (Innovent; *see* WO 2017/024465, WO 2017/025016, WO 2017/132825, and WO 2017/133540), and BCD-100 (Biocad).

In one embodiment, the anti-PD-1 antibody is nivolumab. Nivolumab is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., 2014 Cancer Immunol Res. 2(9):846-56).

In another embodiment, the anti-PD-1 antibody is pembrolizumab. Pembrolizumab is a humanized monoclonal IgG4 (S228P) antibody directed against human cell surface receptor PD-1 (programmed death-1 or programmed cell death-1). Pembrolizumab is described, for example, in U.S. Patent Nos. 8,354,509 and 8,900,587.

Anti-PD-1 antibodies usable in the disclosed compositions and methods also include isolated antibodies that bind specifically to human PD-1 and cross-compete for binding to human PD-1 with any anti-PD-1 antibody disclosed herein, *e.g.*, nivolumab (*see, e.g.,* U.S. Patent No. 8,008,449 and 8,779,105; WO 2013/173223). In some embodiments, the anti-PD-1 antibody binds the same epitope as any of the anti-PD-1 antibodies described herein, *e.g.*, nivolumab. The ability of antibodies to cross-compete for binding to an antigen indicates that these monoclonal antibodies bind to the same epitope region of the antigen and sterically hinder the binding of other cross-competing antibodies to that particular epitope region. These cross-competing antibodies are expected to have functional properties very similar those of the reference antibody, e.g., nivolumab, by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (*see, e.g.,* WO 2013/173223).

In certain embodiments, the antibodies that cross-compete for binding to human PD-1 with, or bind to the same epitope region of human PD-1 antibody, nivolumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-1 antibodies usable in the compositions and methods of the disclosure also include antigen-binding portions of the above antibodies. It has been amply demonstrated that the antigen-binding function of an antibody can be performed by fragments of a full-length antibody.

Anti-PD-1 antibodies suitable for use in the disclosed compositions and methods are antibodies that bind to PD-1 with high specificity and affinity, block the binding of PD-L1 and or PD-L2, and inhibit the immunosuppressive effect of the PD-1 signaling pathway. In any of the compositions or methods disclosed herein, an anti-PD-1 "antibody" includes an antigen-binding portion or fragment that binds to the PD-1 receptor and exhibits the functional properties similar to those of whole antibodies in inhibiting ligand binding and up-regulating the immune system. In certain embodiments, the anti-PD-1 antibody or antigen-binding portion thereof cross-competes with nivolumab for binding to human PD-1.

In some embodiments, the anti-PD-1 antibody is a bispecific antibody. In particular embodiments, the anti-PD-1 antibody is a bispecific antibody that binds both PD-1 and LAG-3.

### 6. Anti-PD-L1 Antibodies

In certain embodiments, the present application encompasses use of an anti-PD-L1 antibody as the PD-1 pathway inhibitor. In one embodiment, the anti-PD-L1 antibody inhibits the binding of PD-L1 receptor, i.e., PD-1 to its ligand PD-L1.

Because anti-PD-1 and anti-PD-L1 target the same signaling pathway and have been shown in clinical trials to exhibit similar levels of efficacy in a variety of cancers, including renal cell carcinoma (*see* Brahmer et al. (2012) N Engl J Med 366:2455-65; Topalian et al. (2012a) N Engl J Med 366:2443-54; WO 2013/173223), an anti-PD-L1 antibody may be substituted for the anti-PD-1 antibody in any of the therapeutic methods disclosed herein. Accordingly, certain aspects of the present disclosure are directed to methods for treating a subject afflicted with a tumor, *e.g.*, SCLC, having a high TMB status comprising administering to the subject an anti-PD-L1 antibody alone ("monotherapy") or an anti-PD-L1 antibody in combination with an anti-CTLA-4 antibody. Anti-PD-L1 antibodies that are known in the art can be used in the compositions and methods of the present disclosure. Examples of anti-PD-L1 antibodies useful in the compositions and methods of the present disclosure include the antibodies disclosed in US Patent No. 9,580,507. Anti-PD-L1 human monoclonal antibodies disclosed in U.S. Patent No. 9,580,507 have been demonstrated to exhibit one or more of the following characteristics: (a) bind to human PD-L1 with a $K_D$ of $1 \times 10^{-7}$ M or less, as determined by surface plasmon resonance using a Biacore biosensor system; (b) increase T-cell proliferation in a Mixed Lymphocyte Reaction (MLR) assay; (c) increase interferon-$\gamma$ production in an MLR assay; (d) increase IL-2 secretion in an MLR assay; (e) stimulate antibody responses; and (f) reverse the effect of T regulatory cells on T cell effector cells and/or dendritic cells. Anti-PD-L1 antibodies usable in the present disclosure include monoclonal antibodies that bind specifically to human PD-L1 and exhibit at least one, in some embodiments, at least five, of the preceding characteristics.

In certain embodiments, the anti-PD-L1 antibody is selected from the group consisting of BMS-936559 (also known as 12A4, MDX-1105; *see, e.g.,* U.S. Patent No. 7,943,743 and WO 2013/173223), atezolizumab (Roche; also known as TECENTRIQ®; MPDL3280A, RG7446; *see* US 8,217,149; *see, also,* Herbst et al. (2013) J Clin Oncol 31(suppl):3000), durvalumab (AstraZeneca; also known as IMFINZI™, MEDI-4736; *see* WO 2011/066389), avelumab (Pfizer; also known as BAVENCIO®, MSB-0010718C; *see* WO 2013/079174), STI-1014 (Sorrento; *see* WO2013/181634), CX-072 (Cytomx; *see* WO2016/149201), KN035 (3D Med/Alphamab; *see* Zhang et al., Cell Discov. 7:3 (March 2017), LY3300054 (Eli Lilly Co.; *see, e.g.,* WO 2017/034916), and CK-301 (Checkpoint Therapeutics; *see* Gorelik et al., AACR:Abstract 4606 (Apr 2016)).

In certain embodiments, the PD-L1 antibody is atezolizumab (TECENTRIQ®). Atezolizumab is a fully humanized IgG1 monoclonal anti-PD-L1 antibody.

In certain embodiments, the PD-L1 antibody is durvalumab (IMFINZI™). Durvalumab is a human IgG1 kappa monoclonal anti-PD-L1 antibody.

In certain embodiments, the PD-L1 antibody is avelumab (BAVENCIO®). Avelumab is a human IgG 1 lambda monoclonal anti-PD-L1 antibody.

Anti-PD-L1 antibodies usable in the disclosed compositions and methods also include isolated antibodies that bind specifically to human PD-L1 and cross-compete for binding to human PD-L1 with any anti-PD-L1 antibody disclosed herein, *e.g.*, atezolizumab, durvalumab, and/or avelumab. In some embodiments, the anti-PD-L1 antibody binds the same epitope as any of the anti-PD-L1 antibodies described herein, *e.g.*, atezolizumab, durvalumab, and/or avelumab. The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar those of the reference antibody, *e.g.*, atezolizumab and/or avelumab, by virtue of their binding to the same epitope region of PD-L1. Cross-competing antibodies can be readily identified based on their ability to cross-compete with atezolizumab and/or avelumab in standard PD-L1 binding assays such as Biacore analysis, ELISA assays or flow cytometry (*see, e.g.,* WO 2013/173223).

In certain embodiments, the antibodies that cross-compete for binding to human PD-L1 with, or bind to the same epitope region of human PD-L1 antibody as, atezolizumab, durvalumab, and/or avelumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-L1 antibodies usable in the compositions and methods of the disclosed disclosure also include antigen-binding portions of the above antibodies. It has been amply demonstrated that the antigen-binding function of an antibody can be performed by fragments of a full-length antibody.

Anti-PD-L1 antibodies suitable for use in the disclosed compositions and methods are antibodies that bind to PD-L1 with high specificity and affinity, block the binding of PD-1, and inhibit the immunosuppressive effect of the PD-1 signaling pathway. In any of the compositions or methods disclosed herein, an anti-PD-L1 "antibody" includes an antigen-binding portion or fragment that binds to PD-L1 and exhibits the functional properties similar to those of whole antibodies in inhibiting receptor binding and up-regulating the immune system. In certain embodiments, the anti-PD-L1 antibody or antigen-binding portion thereof cross-competes with atezolizumab, durvalumab, and/or avelumab for binding to human PD-L1.

The anti-PD-L1 antibody useful for the present disclosure can be any PD-L1 antibody that specifically binds to PD-L1, *e.g.*, antibodies that cross-compete with durvalumab, avelumab, or atezolizumab for binding to human PD-1, *e.g.*, an antibody that binds to the same epitope as durvalumab, avelumab, or atezolizumab. In a particular embodiment, the anti-PD-L1 antibody is durvalumab. In other embodiments, the anti-PD-L1 antibody is avelumab. In some embodiments, the anti-PD-L1 antibody is atezolizumab.

## 7. Anti-CTLA-4 Antibodies

In certain embodiments, the present application encompasses use of an anti-CTLA-4 antibody. In one embodiment, the anti-CTLA-4 antibody binds to and inhibits CTLA-4. In some embodiments, the anti-CTLA-4 antibody is ipilimumab (YERVOY), tremelimumab (ticilimumab; CP-675,206), AGEN-1884, or ATOR-1015.

## 8. Pharmaceutical Compositions

Pharmaceutical compositions suitable for administration to human patients are typically formulated for parenteral administration, e.g., in a liquid carrier, or suitable for reconstitution into liquid solution or suspension for intravenous administration.

In general, such compositions typically comprise a pharmaceutically acceptable carrier. As used herein, the term "pharmaceutically acceptable" means approved by a government regulatory agency or listed in the U.S. Pharmacopeia or another generally recognized pharmacopeia for use in animals, particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil, glycerol polyethylene glycol ricinoleate, and the like. Water or aqueous solution saline and aqueous dextrose and glycerol solutions may be employed as carriers, particularly for injectable solutions (e.g., comprising an anti-LAG-3 and anti-PD-1 antibody). Liquid compositions for parenteral administration can be formulated for administration by injection or continuous infusion. Routes of administration by injection or infusion include intravenous, intraperitoneal, intramuscular, intrathecal and subcutaneous. In one embodiment, the anti-LAG-3 and anti-PD-1 antibodies are administered intravenously (e.g., in separate formulations or together (in the same formulation or in separate formulations)).

## 9. Patient Populations

Provided herein are clinical methods for metastatic or unresectable melanoma in a human patient using an immunotherapy disclosed herein, for example, a combination of a LAG-3 inhibitor (e.g., an anti-LAG-3 antibody) and a PD-1 pathway inhibitor (e.g., an anti-PD-1 antibody). In certain embodiments, the patient who receives the immunotherapy disclosed herein is treatment naive in the metastatic setting. In one embodiment, the human patient has not received previous therapy for metastatic or unresectable melanoma. In certain embodiments, the patient has not received previous systemic therapies. In some embodiments, the administered therapies of the present invention are first-line therapies in the metastatic setting. In some embodiments, the melanoma is a previously untreated metastatic or unresectable melanoma.

In certain embodiments, the patient received prior treatment when the melanoma was locally advanced. In an embodiment, the human patient received prior adjuvant melanoma therapy. In one embodiment, the prior adjuvant melanoma therapy was an anti-PD-1 therapy. In a different embodiment, the prior adjuvant melanoma therapy was an anti-CTL-4 therapy. In certain embodiments, the human patient received prior neoadjuvant melanoma therapy. In some embodiments, the patient received the therapy in a time period more than 6 months before the date of recurrence. In a further embodiment, the patient received adjuvant interferon therapy. In another embodiment, the patient received neoadjuvant interferon therapy. In one embodiment, the patient received BRAF-inhibitor containing therapy. In certain embodiments, the patient received or MEK-inhibitor containing therapy. In one embodiment, the patient received the prior adjuvant melanoma therapy in a time period greater than or equal to 6 months between the last dose and the date of recurrence. In one embodiment, the patient suffers from metastatic or unresectable melanoma that is refractory to treatment with a cancer therapy. In some embodiments, the cancer therapy can be radiation therapy, surgery, chemotherapy, gene therapy, DNA therapy, viral therapy, RNA therapy, immunotherapy, bone marrow transplantation, nanotherapy, monoclonal antibody therapy, or a combination of the foregoing. The therapy may be in the form of adjuvant or neoadjuvant therapy. "Adjuvant therapy," as used herein refers to cancer treatment given after the primary treatment to lower the risk that the cancer will come back. Adjuvant therapy may include chemotherapy, radiation therapy, hormone therapy, targeted therapy, or biological therapy. Adjuvant therapy is often used after primary treatments, such as surgery or radiation. Adjuvant therapy given before the main treatment is called neoadjuvant therapy. This type of adjuvant therapy can also decrease the chance of the cancer coming back, and it is often used to make the primary treatment, e.g., surgery or radiation treatment, more effective in reducing tumor burden. In another embodiment, the patient suffers from metastatic or unresectable melanoma that is refractory to treatment with chemotherapy.

In another embodiment, the patient suffers from metastatic or unresectable melanoma that is refractory to treatment with an immune checkpoint inhibitor. In another embodiment, the patient suffers from metastatic or unresectable melanoma that is refractory to treatment with a PD-1 inhibitor. In another embodiment, the patient suffers from metastatic or unresectable melanoma that is refractory to treatment with an anti-PD-1 antibody. In one embodiment, the patient suffers from metastatic or unresectable melanoma that is predicted to be refractory to treatment with an anti-PD-1 antibody. In one embodiment, the metastatic or unresectable melanoma is deemed refractory to anti-PD-1 therapy based on biomarker analysis. In certain embodiments, the metastatic or unresectable melanoma is refractory to PD-1 monotherapy. In another embodiment, the patient suffers from metastatic or unresectable melanoma that is refractory to treatment with an anti-PD-L1 antibody.

Patients can be tested or selected for one or more of the above described clinical attributes prior to, during or after treatment.

In some embodiments, the patient is an adolescent 12-17 years of age.

## 10. Immunotherapies

In one aspect, immunotherapies provided herein involve administration of a LAG-3 inhibitor (e.g., an anti-LAG-3 antibody) and a PD-1 pathway inhibitor (e.g., an anti-PD-1 antibody or an anti-PD-L1 antibody) to treat metastatic or unresectable melanoma.

In one embodiment, the invention provides an anti-LAG-3 antibody and an anti-PD-1 antibody according to a defined clinical dosage regimen, to treat subjects having metastatic or unresectable melanoma. In a particular embodiment, the anti-LAG-3 antibody is BMS-986016. In another embodiment, the anti-PD-1 antibody is BMS-936558. In another embodiment, dosage regimens are fixed. In another embodiment, dosage regimens are adjusted to provide the optimum desired response (e.g., an effective response). In one embodiment, the anti-LAG-3 antibody and anti-PD-1 antibody are administered at 160 mg anti-LAG-3 antibody and 480 mg of anti-PD-1 antibody. In one embodiment, the anti-LAG-3 antibody and anti-PD-1 antibody are administered at 80 mg anti-LAG-3 antibody and 240 mg of anti-PD-1 antibody. In an embodiment, the anti-LAG-3 antibody and anti-PD-1 antibody are administered at 160 mg anti-LAG-3 antibody and 240 mg of anti-PD-1 antibody. In some embodiments, the anti-LAG-3 antibody and anti-PD-1 antibody are administered at 240 mg anti-LAG-3 antibody and 240 mg of anti-PD-1 antibody. In one embodiment, the anti-LAG-3 antibody and anti-PD-1 antibody are administered at 480 mg anti-LAG-3 antibody and 480 mg of anti-PD-1 antibody. In certain embodiments, the ratio of anti-LAG-3 antibody to anti-PD-1 antibody is 1:1, 1:2, 1:3, 1:4, 1:5, 1:6 , 2:1, 3:1, 3.5:1, 4:1, 5:1, or 6:1.

As used herein, adjunctive or combined administration (coadministration) includes simultaneous administration of the compounds in the same or different dosage form, or separate administration of the compounds (e.g., sequential administration). Thus, for example, the anti-LAG-3 and anti-PD-1 antibodies can be simultaneously administered in a single formulation. Alternatively, the anti-LAG-3 and anti-PD-1 antibodies can be formulated for separate administration and are administered concurrently or sequentially (e.g., one antibody is administered within about 30 minutes prior to administration of the second antibody).

For example, the anti-PD-1 antibody can be administered first followed by (e.g., immediately followed by) the administration of the anti-LAG-3 antibody, or vice versa. In one embodiment, the anti-PD-1 antibody is administered prior to administration of the anti-LAG-3 antibody. In another embodiment, the anti-PD-1 antibody is administered after administration of the anti-LAG-3 antibody. In another embodiment, the anti-LAG-3 antibody and anti-PD-1 antibody are administered concurrently. Such concurrent or sequential administration preferably results in both antibodies being simultaneously present in treated patients.

## 11. Treatment Protocols

In one aspect, suitable treatment protocols for treating a malignant tumor in a human patient include administering to the patient an effective amount of a LAG3 inhibitor (e.g., an anti-LAG-3 antibody) and a PD-1 pathway inhibitor (e.g., an anti-PD-1 antibody).

In some embodiments, a suitable treatment protocol for treating a malignant tumor in a human patient include, for example, administering to the patient an effective amount of each of:

(a) an anti-LAG-3 antibody, such as one comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and
(b) an anti-PD-1 antibody, such as one comprising CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21;

wherein the method comprises at least one administration cycle, wherein the cycle is a period of 4 weeks, wherein for each of the at least one cycles, at least one dose of the anti-LAG-3 antibody is administered at a dose of about 1, about 3, about 10, about 20, about 50, about 80, about 100, about 120, about 130, about 150, about 160, about 180, about 200, about 240, about 280, about 300, about 320, about 360, about 400, about 440, about 480, or about 500 mg and at least one dose of the anti-PD-1 antibody is administered at a dose of about 50, about 80, about 100, about 130, about 150, about 180, about 200, about 240, about 280, about 320, about 360, about 400, about 440, about 480, about 500, about 520, about 560, about 600, about 650, about 700 or about 800 mg. In another embodiment, one dose of the anti-LAG-3 antibody is administered at a dose of 0.01, 0.03, 0.25, 0.1, 0.3, 1 or 2, 3, 5, 8 or 10 mg/kg body weight and one dose of the anti-PD-1 antibody are administered at a dose of 0.1, 0.3, 1, 2, 3, 4, 5, 6, 7, 8, 9 or 10 mg/kg body weight.

In one embodiment, the anti-LAG-3 antibody and anti-PD-1 antibody are administered at 160 mg anti-LAG-3 antibody and 480 mg of anti-PD-1 antibody.

In one embodiment, the tumor is metastatic or unresectable melanoma. In a further embodiment the tumor is previously untreated metastatic or unresectable melanoma.

In one embodiment, the method provides a method of inhibiting the growth of a metastatic melanoma tumor in a human patient, the method comprising administering to the patient an effective amount of: (a) 160 mg of anti-LAG-3 antibody; and (b) 480 mg of an anti-PD-1 antibody wherein the patient has not received prior treatment for metastatic melanoma (i.e., wherein the treatment is a first-line treatment). In some embodiments, the patient has not received prior systemic treatment. In certain embodiments, the patient has not received prior surgery for melanoma. In another embodiment, the patient has not received radiation. In an embodiment, the an anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and the anti-PD-1 antibody comprises CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21. In certain embodiments, the anti-LAG-3 antibody is relatlimab and the anti-PD-1 antibody is nivolumab. In one embodiment, a cycle of administration is four weeks (Q4W), which can be repeated, as necessary.

In one embodiment, the method provides a method of inhibiting the growth of an unresectable melanoma tumor in a human patient, the method comprising administering to the patient an effective amount of: (a) 160 mg of anti-LAG-3 antibody; and (b) 480 mg of an anti-PD-1 antibody wherein the patient has not received prior treatment for unresectable melanoma (i.e., wherein the treatment is a first-line treatment). In some embodiments, the patient has not received prior systemic treatment. In certain embodiments, the patient has not received prior surgery for melanoma. In another embodiment, the patient has not received radiation. In an embodiment, the an anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and the anti-PD-1 antibody comprises CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21. In certain embodiments, the anti-LAG-3 antibody is relatlimab and the anti-PD-1 antibody is nivolumab. In one embodiment, a cycle of administration is four weeks (Q4W), which can be repeated, as necessary.

In one embodiment, the method provides a method of treating a metastatic melanoma tumor in a human patient, the method comprising administering to the patient an effective amount of: (a) a LAG-3 antagonist; and (b) a PD-1 pathway inhibitor; wherein the patient has not received prior treatment for metastatic melanoma (i.e., wherein the treatment is a first-line treatment). In some embodiments, the patient has not received prior systemic treatment. In certain embodiments, the patient has not received prior surgery for melanoma. In another embodiment, the patient has not received radiation. In one embodiment, the anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and the anti-PD-1 antibody comprises CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21. In certain embodiments, the anti-LAG-3 antibody is relatlimab and the anti-PD-1 antibody is nivolumab. In one embodiment, a cycle of administration is four weeks (Q4W), which can be repeated, as necessary.

In one embodiment, the method provides a method of treating an unresectable melanoma tumor in a human patient, the method comprising administering to the patient an effective amount of: (a) a LAG-3 antagonist; and (b) a PD-1 pathway inhibitor; wherein the patient has not received prior treatment for unresectable melanoma (i.e., wherein the treatment is a first-line treatment). In some embodiments, the patient has not received prior systemic treatment. In certain embodiments, the patient has not received prior surgery for melanoma. In another embodiment, the patient has not received radiation. In some embodiments, the an anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and the anti-PD-1 antibody comprises CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21. In certain embodiments, the anti-LAG-3 antibody is relatlimab and the anti-PD-1 antibody is nivolumab. In one embodiment, a cycle of administration is four weeks (Q4W), which can be repeated, as necessary.

In another embodiment, the amount of the anti-LAG-3 and/or anti-PD-1 antibodies administered is constant for each dose. In another embodiment, the amount of antibody administered varies with each dose. For example, the maintenance (or follow-on) dose of the antibody can be higher or the same as the loading dose which is first administered. In another embodiment, the maintenance dose of the antibody can be lower or the same as the loading dose.

In another embodiment, the anti-LAG-3 and anti-PD-1 antibodies are formulated for intravenous administration. In one embodiment, the anti-LAG-3 antibody and anti-PD-1 antibody are administered on Days 1 and 28 of each cycle.

In another embodiment, a cycle of administration is four weeks, which can be repeated, as necessary.

In one embodiment, the anti-LAG-3 antibody is BMS-986016 and the anti-PD-1 antibody is nivolumab. In one embodiment, the anti-LAG-3 antibody is MK-4280 and the anti-PD-1 antibody is pembrolizumab. In one embodiment, the anti-LAG-3 antibody is REGN3767 and the anti-PD-1 antibody is REGN2810. In one embodiment, the anti-LAG-3 antibody is LAG525 and the anti-PD-1 antibody is PDR001. In some embodiments, the anti-PD-1 antibody is a bispecific antibody. In some embodiments, the anti-LAG-3-1 antibody is a bispecific antibody. In particular embodiments, the bispecific antibody binds both PD-1 and LAG-3. In one embodiment, the PD-1/LAG-3 bispecific antibody is TSR-075. In some embodiments, the PD-1/LAG-3 bispecific antibody is MGD013. In particular embodiments, the antibody is a PD-L1/LAG-3 bispecific antibody. In some embodiments, the PD-L1/LAG-3 bispecific antibody is FS-118.

In another aspect, the invention features any of the aforementioned embodiments, wherein the anti-PD-1 antibody is replaced by, or combined with, an anti-PD-L1 or anti-PD-L2 antibody.

## 12. Outcomes

A patient treated according to the methods disclosed herein preferably experience improvement in at least one sign of cancer. In one embodiment, improvement is measured by a reduction in the quantity and/or size of measurable tumor lesions. In another embodiment, lesions can be measured on chest x-rays or CT or MRI films. In another embodiment, cytology or histology can be used to evaluate responsiveness to a therapy.

In one embodiment, the patient treated exhibits a complete response (CR), a partial response (PR), stable disease (SD), immune-related complete disease (irCR), immune-related partial response (irPR), or immune-related stable disease (irSD). In another embodiment, the patient treated experiences tumor shrinkage and/or decrease in growth rate, i.e., suppression of tumor growth. In another embodiment, unwanted cell proliferation is reduced or inhibited. In yet another embodiment, one or more of the following can occur: the number of cancer cells can be reduced; tumor size can be reduced; cancer cell infiltration into peripheral organs can be inhibited, retarded, slowed, or stopped; tumor metastasis can be slowed or inhibited; tumor growth can be inhibited; recurrence of tumor can be prevented or delayed; one or more of the symptoms associated with cancer can be relieved to some extent.

In other embodiments, administration of effective amounts of the anti-LAG-3 antibody and anti-PD-1 antibody according to any of the methods provided herein produces at least one therapeutic effect selected from the group consisting of reduction in size of a tumor, reduction in number of metastatic lesions appearing over time, complete remission, partial remission, or stable disease.

In still other embodiments, the methods of treatment produce a clinical benefit rate (CBR=CR+PR+SD$\geq$6 months) better than that achieved by a method of treatment that does not comprise a step of (i) determining the level of LAG-3, PD-L1 and/or BRAF V600 expression in a tumor sample prior to treatment, and (ii) treating the tumor. In other embodiments, the improvement of clinical benefit rate is about 20% 20%, 30%, 40%, 50%, 60%, 70%, 80% or more compared to a method of treatment that does not comprise a step of (i) determining the level of LAG-3, PD-L1 and/or BRAF V600 expression in a tumor sample prior to treatment, and (ii) treating the tumor. In still other embodiments, the methods of treatment produce an objective response rate (ORR=CR+PR) of at least about 15%, at least about 20%, at least about 25%, at least about 30%, at least about 40%, at least about 50%, at least about 60%, at least about 70%, at least about 80%, at least about 90%, or about 100%.

### 13. Kits and Unit Dosage Forms

Also within the scope of the present invention are diagnostic kits comprising an anti-LAG-3 antibody for assaying LAG-3 expression as a biomarker for screening patients for the immunotherapy or for predicting the efficacy of the immunotherapy. Kits typically include a label indicating the intended use of the contents of the kit and instructions for use. The term "label" includes any writing, or recorded material supplied on or with the kit, or which otherwise accompanies the kit. In certain embodiments of a diagnostic kit, a first anti-LAG-3 antibody for assaying, detecting, and/or quantifying LAG-3 expression is co-packaged with at least one therapeutic antibody (e.g., a second anti-LAG-3 antibody and an anti-PD-1 antibody) for the treatment of a metastatic or unresectable melanoma. In some embodiments, the kit further comprises an anti-PD-L1 antibody for assaying, detecting, and/or quantifying PD-L1 expression as a biomarker for predicting the efficacy of the immunotherapy. In one embodiment, the immunotherapy comprises administering to the patient a therapeutically effective amount of a LAG-3 inhibitor (e.g., anti-LAG-3 antibody) and a PD-1 pathway inhibitor (e.g., anti-PD1 antibody).

In certain embodiments, the diagnostic kit comprises an anti-human LAG-3 monoclonal antibody for assaying, detecting, and/or quantifying LAG-3 expression. See, e.g., J. Matsuzaki, et al.; PNAS 107, 7875 (2010).

Also provided herein are therapeutic kits which include a pharmaceutical composition containing an anti-LAG-3 antibody, such as BMS-986016, and an anti-PD-1 antibody, such as nivolumab, in a therapeutically effective amount adapted for use in the preceding methods. In certain embodiments of a therapeutic kit, the anti-LAG-3 antibody is co-packaged with an anti-PD-1 antibody in unit dosage form. The kits optionally also can include instructions, e.g., comprising administration schedules, to allow a practitioner (e.g., a physician, nurse, or patient) to administer the composition contained therein to administer the composition to a patient having cancer (e.g., a solid tumor). The kit also can include a syringe.

Optionally, the diagnostic and/or therapeutic kits include multiple packages of the single-dose pharmaceutical compositions each containing an effective amount of the anti-LAG-3 or anti-PD-1 antibody for a single administration in accordance with the methods provided above. Instruments or devices necessary for administering the pharmaceutical composition(s) also may be included in the kits. For instance, a kit may provide one or more pre-filled syringes containing an amount of the anti-LAG-3 or anti-PD-1 antibody.

In one embodiment, the present invention provides a kit for treating a patient afflicted with metastatic or unresectable melanoma, the kit, for example, comprising:

(a) a dose of an anti-LAG-3 antibody, such as one comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5;
(b) a dose of an anti-PD-1 antibody, such as one comprising CDR1, CDR2 and CDR3 domains of the heavy chain variable region having the sequence set forth in SEQ ID NO:19, and CDR1, CDR2 and CDR3 domains of the light chain variable region having the sequence set forth in SEQ ID NO:21; and
(c) instructions for using the anti-LAG-3 antibody and anti-PD-1 antibody in the methods described herein.

In some embodiments, the malignant tumor is metastatic or unresectable melanoma.

The present invention is further illustrated by the following examples which should not be construed as further limiting. The contents of all references cited throughout this application are expressly incorporated herein by reference.

EXAMPLES

**EXAMPLE 1**

Efficacy of Anti-Lymphocyte Activation Gene-3 Antibody (Anti-LAG-3; BMS-986016) in Combination With Nivolumab in Patients With Previously Untreated Metastatic or Unresectable Melanoma

The purpose of this study is to evaluate the combination of BMS-986016 (relatlimab) and nivolumab (BMS936558) in the treatment of metastatic or unresectable melanoma.

Patients are selected based on the following eligibility criteria: (1) having metastatic or unresectable melanoma; (2) no prior systemic therapy for metastatic or unresectable disease; (3) Eastern Cooperative Oncology Group PS 0-1; (4) tumor tissue available for biomarker analyses. Patients are stratified by: (1) tumor cell PD-L1 expression ($\geq$ 1% or < 1%) determined using Dako PD-L1 IHC 28-8 kit; (2) immune cell LAG-3 expression ($\geq$ 1% or < 1%) determined using mouse antibody clone 17B4; (3) BRAF V600 mutant versus wild-type BRAF expression; and (4) American Joint Committee on Cancer (8th Edition) stage.

During the treatment phase, adult patients will receive BMS-986016 (relatlimab) 160 mg and nivolumab 480 mg for each treatment cycle every four weeks. Adolescent patients aged 12-17 must also have a Lansky performance status of greater than or equal to 80%. Adolescent patients less than 40 kg will receive BMS-986016 (relatlimab) at a dose of 2 mg/kg and nivolumab at a dose of 6 mg/kg for each treatment cycle every four weeks.

SEQUENCES

SEQ ID NO:1 Heavy Chain Amino Acid Sequence; Anti-LAG-3 mAb (BMS-986016)

```
QVQLQQWGAGLLKPSETLSLTCAVYGGSFSDYYWNWIRQPPGKGLEWIGEINHRGSTNSNPSLKS
RVTLSLDTSKNQFSLKLRSVTAADTAVYYCAFGYSDYEYNWFDPWGQGTLVTVSSASTKGPSVFP
LAPCSRSTSESTAALGCLVKDYFPEPVTVSWNSGALTSGVHTFPAVLQSSGLYSLSSVVTVPSSS
LGTKTYTCNVDHKPSNTKVDKRVESKYGPPCPPCPAPEFLGGPSVFLFPPKPKDTLMISRTPEVT
CVVVDVSQEDPEVQFNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLGK
```

SEQ ID NO:2 Light Chain Amino Acid Sequence; Anti-LAG-3 mAb (BMS-986016)

```
EIVLTQSPATLSLSPGERATLSCRASQSISSYLAWYQQKPGQAPRLLIYDASNRATGIPARFSGS
GSGTDFTLTISSLEPEDFAVYYCQQRSNWPLTFGQGTNLEIKRTVAAPSVFIFPPSDEQLKSGTA
SVVCLLNNFYPREAKVQWKVDNALQSGNSQESVTEQDSKDSTYSLSSTLTLSKADYEKHKVYACE
VTHQGLSSPVTKSFNRGEC
```

SEQ ID NO:3 Heavy Chain Variable Region (VH) Amino Acid Sequence; Anti-LAG-3 mAb (BMS-986016)

```
QVQLQQWGAGLLKPSETLSLTCAVYGGSFSDYYWNWIRQPPGKGLEWIGEINHRGSTNSNPSLKS
RVTLSLDTSKNQFSLKLRSVTAADTAVYYCAFGYSDYEYNWFDPWGQGTLVTVSS
```

SEQ ID NO:4 Heavy Chain Variable Region (VH) Nucleotide Sequence; Anti-LAG-3 mAb (BMS-986016)

```
caggtgcagctacagcagtggggcgcaggactgttgaagccttcggagaccctgtccctcacctg
cgctgtctatggtgggtccttcagtgattactactggaactggatccgccagccccagggaagg
ggctggagtggattggggaaatcaatcatcgtggaagcaccaactccaacccgtccctcaagagt
cgagtcaccctatcactagacacgtccaagaaccagttctccctgaagctgaggtctgtgaccgc
cgcggacacggctgtgtattactgtgcgtttggatatagtgactacgagtacaactggttcgacc
cctggggccagggaaccctggtcaccgtctcctca
```

SEQ ID NO:5 Light Chain Variable Region (VL) Amino Acid Sequence; Anti-LAG-3 mAb (BMS-986016)

```
EIVLTQSPATLSLSPGERATLSCRASQSISSYLAWYQQKPGQAPRLLIYDASNRATGIPARFSGS
GSGTDFTLTISSLEPEDFAVYYCQQRSNWPLTFGQGTNLEIK
```

SEQ ID NO:6 Light Chain Variable Region (VL) Nucleotide Sequence; Anti-LAG-3 mAb (BMS-986016)

```
gaaattgtgttgacacagtctccagccaccctgtctttgtctccaggggaaagagccaccctctc
ctgcagggccagtcagagtattagcagctacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagcgtagcaactggcctctcacttttggccaggggaccaacctggagatcaaa
```

SEQ ID NO:7 Heavy Chain CDR1 Amino Acid Sequence; Anti-LAG-3 mAb (BMS-986016)
DYYWN

SEQ ID NO:8 Heavy Chain CDR2 Amino Acid Sequence; Anti-LAG-3 mAb (BMS-986016)
EINHRGSTNSNPSLKS

SEQ ID NO:9 Heavy Chain CDR3 Amino Acid Sequence; Anti-LAG-3 mAb (BMS-986016)
GYSDYEYNWEDP

SEQ ID NO:10 Light Chain CDR1 Amino Acid Sequence; Anti-LAG-3 mAb (BMS-986016)
RASQSISSYLA

SEQ ID NO:11 Light Chain CDR2 Amino Acid Sequence; Anti-LAG-3 mAb (BMS-986016)
DASNRAT

SEQ ID NO:12 Light Chain CDR3 Amino Acid Sequence; Anti-LAG-3 mAb (BMS-986016)
QQRSNWPLT

SEQ ID NO:13 Heavy Chain Amino Acid Sequence; Anti-PD-1 mAb (BMS936558)

```
QVQLVESGGGVVQPGRSLRLDCKASGITFSNSGMHWVRQAPGKGLEWVAVIWYDGSKRYYADSVK
GRFTISRDNSKNTLFLQMNSLRAEDTAVYYCATNDDYWGQGTLVTVSSASTKGPSVFPLAPCSRS
TSESTAALGCLVKDYFPEPVTVSWNSGALTSGVHTFPAVLQSSGLYSLSSVVTVPSSSLGTKTYT
CNVDHKPSNTKVDKRVESKYGPPCPPCPAPEFLGGPSVFLFPPKPKDTLMISRTPEVTCVVVDVS
QEDPEVQFNWYVDGVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGK
```

SEQ ID NO:14 Light Chain Amino Acid Sequence; Anti-PD-1 mAb (BMS936558)

```
EIVLTQSPATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIKRTVAAPSVFIFPPSDEQLKSGTA
SVVCLLNNFYPREAKVQWKVDNALQSGNSQESVTEQDSKDSTYSLSSTLLSKADYEKHKVYACEV
THQGLSSPVTKSFNRGEC
```

SEQ ID NO:15 Heavy Chain Variable Region (VH) Amino Acid Sequence; Anti-PD-1 mAb (BMS936558)

```
QVQLVESGGGVVQPGRSLRLDCKASGITFSNSGMHWVRQAPGKGLEWVAVIWYDGSKRYYADSVK
GRFTISRDNSKNTLFLQMNSLRAEDTAVYYCATNDDYWGQGTLVTVSS
```

SEQ ID NO:16 Heavy Chain Variable Region (VH) Nucleotide Sequence; Anti-PD-1 mAb (BMS936558)

```
caggtgcagctggtggagtctggggggaggcgtggtccagcctggggaggtccctgagactcgactg
taaagcgtctggaatcaccttcagtaactctggcatgcactgggtccgccaggctccaggcaagg
ggctggagtgggtggcagttatttggtatgatggaagtaaaagatactatgcagactccgtgaag
ggccgattcaccatctccagagacaattccaagaacacgctgtttctgcaaatgaacagcctgag
agccgaggacacggctgtgtattactgtgcgacaaacgacgactactggggccagggaaccctgg
tcaccgtctcctca
```

SEQ ID NO:17 Light Chain Variable Region (VL) Amino Acid Sequence; Anti-PD-1 mAb (BMS936558)

```
EIVLTQSPATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIK
```

SEQ ID NO:18 Light Chain Variable Region (VL) Nucleotide Sequence; Anti-PD-1 mAb (BMS936558)

```
gaaattgtgttgacacagtctccagccaccctgtctttgtctccaggggaaagagccaccctctc
ctgcagggccagtcagagtgttagtagttacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagagtagcaactggcctcggacgttcggccaagggaccaaggtggaaatcaaa
```

SEQ ID NO:19 Heavy Chain CDR1 Amino Acid Sequence; Anti-PD-1 mAb (BMS936558)
NSGMH

SEQ ID NO:20 Heavy Chain CDR2 Amino Acid Sequence; Anti-PD-1 mAb (BMS936558)
VIWYDGSKRYYADSVKG

SEQ ID NO:21 Heavy Chain CDR3 Amino Acid Sequence; Anti-PD-1 mAb (BMS936558)
NDDY

SEQ ID NO:22 Light Chain CDR1 Amino Acid Sequence; Anti-PD-1 mAb (BMS936558)
RASQSVSSYLA

SEQ ID NO:23 Light Chain CDR2 Amino Acid Sequence; Anti-PD-1 mAb (BMS936558)
DASNRAT

SEQ ID NO:24 Light Chain CDR3 Amino Acid Sequence; Anti-PD-1 mAb (BMS936558)
QQSSNWPRT

SEQ ID NO:25 Heavy Chain Nucleotide Sequence; Anti-LAG-3 mAb (BMS-986016)

```
caggtgcagctacagcagtggggcgcaggactgttgaagccttcggagaccctgtccctcacctg
cgctgtctatggtgggtccttcagtgattactactggaactggatccgccagcccccagggaagg
ggctggagtggattggggaaatcaatcatcgtggaagcaccaactccaacccgtccctcaagagt
```

```
cgagtcaccctatcactagacacgtccaagaaccagttctccctgaagctgaggtctgtgaccgc
cgcggacacggctgtgtattactgtgcgtttggatatagtgactacgagtacaactggttcgacc
cctggggccagggaaccctggtcaccgtctcctcagctagcaccaagggcccatccgtcttcccc
ctggcgccctgctccaggagcacctccgagagcacagccgccctgggctgcctggtcaaggacta
cttccccgaaccggtgacggtgtcgtggaactcaggcgccctgaccagcggcgtgcacaccttcc
cggctgtcctacagtcctcaggactctactccctcagcagcgtggtgaccgtgccctccagcagc
ttgggcacgaagacctacacctgcaacgtagatcacaagcccagcaacaccaaggtggacaagag
agttgagtccaaatatggtccccatgcccaccatgcccagcacctgagttcctggggggaccat
cagtcttcctgttccccccaaaacccaaggacactctcatgatctcccggacccctgaggtcacg
tgcgtggtggtggacgtgagccaggaagaccccgaggtccagttcaactggtacgtggatggcgt
ggaggtgcataatgccaagacaaagccgcgggaggagcagttcaacagcacgtaccgtgtggtca
gcgtcctcaccgtcctgcaccaggactggctgaacggcaaggagtacaagtgcaaggtctccaac
aaaggcctcccgtcctccatcgagaaaaccatctccaaagccaaagggcagccccgagagccaca
ggtgtacaccctgcccccatcccaggaggagatgaccaagaaccaggtcagcctgacctgcctgg
tcaaaggcttctaccccagcgacatcgccgtggagtgggagagcaatgggcagccggagaacaac
tacaagaccacgcctcccgtgctggactccgacggctccttcttcctctacagcaggctaaccgt
ggacaagagcaggtggcaggaggggaatgtcttctcatgctccgtgatgcatgaggctctgcaca
accactacacacagaagagcctctccctgtctctgggtaaatga
```

SEQ ID NO:26 Light Chain Nucleotide Sequence; Anti-LAG-3 mAb (BMS-986016)

```
Gaaattgtgttgacacagtctccagccaccctgtctttgtctccaggggaaagagccaccctctc
ctgcagggccagtcagagtattagcagctacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagcgtagcaactggcctctcacttttggccaggggaccaacctggagatcaaacgta
cggtggctgcaccatctgtcttcatcttcccgccatctgatgagcagttgaaatctggaactgcc
tctgttgtgtgcctgctgaataacttctatcccagagaggccaaagtacagtggaaggtggataa
cgccctccaatcgggtaactcccaggagagtgtcacagagcaggacagcaaggacagcacctaca
gcctcagcagcaccctgacgctgagcaaagcagactacgagaaacacaaagtctacgcctgcgaa
gtcacccatcagggcctgagctcgcccgtcacaaagagcttcaacaggggagagtgttag
```

SEQ ID NO: 27 LAG-3 epitope
PGHPLAPG

SEQ ID NO: 28 LAG-3 epitope
HPAAPSSW

SEQ ID NO: 29 LAG-3 epitope
PAAPSSWG

SEQ ID NO:30 Heavy Chain Amino Acid Sequence; Anti-LAG-3 mAb (BMS-986016) without terminal lysine

```
QVQLQQWGAGLLKPSETLSLTCAVYGGSFSDYYWNWIRQPPGKGLEWIGEINHRGSTNSNPSLKS
RVTLSLDTSKNQFSLKLRSVTAADTAVYYCAFGYSDYEYNWFDPWGQGTLVTVSSASTKGPSVFP
LAPCSRSTSESTAALGCLVKDYFPEPVTVSWNSGALTSGVHTFPAVLQSSGLYSLSSVVTVPSSS

LGTKTYTCNVDHKPSNTKVDKRVESKYGPPCPPCPAPEFLGGPSVFLFPPKPKDTLMISRTPEVT
CVVVDVSQEDPEVQFNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLG
```

The following paragraphs set out further aspects of the present invention.

1. A method of inhibiting the growth of a metastatic melanoma tumor in a human patient, the method comprising administering to the patient an effective amount of:

(a) a LAG-3 antagonist; and
(b) a PD-1 pathway inhibitor;

wherein the patient has not received prior systemic treatment for metastatic melanoma.

2. A method of treating a metastatic melanoma tumor in a human patient, the method comprising administering to the patient an effective amount of:

(a) a LAG-3 antagonist; and
(b) a PD-1 pathway inhibitor;

wherein the patient has not received prior systemic treatment for metastatic melanoma.

3. A method of inhibiting the growth of an unresectable melanoma tumor in a human patient, the method comprising administering to the patient an effective amount of:

(a) a LAG-3 antagonist; and
(b) a PD-1 pathway inhibitor;

wherein the patient has not received prior systemic treatment for unresectable melanoma.

4. A method of treating an unresectable melanoma tumor in a human patient, the method comprising administering to the patient an effective amount of:

(a) a LAG-3 antagonist; and
(b) a PD-1 pathway inhibitor;

wherein the patient has not received prior systemic treatment for unresectable melanoma.

5. The method of any one of paragraphs 1-4, wherein the patient received prior treatment when the melanoma was locally advanced, wherein the prior treatment was adjuvant or neoadjuvant anti-PD-1 or anti-CTLA-4 therapy.

6. The method of any one of paragraphs 3 or 4, wherein the patient has histologically confirmed unresectable stage III or IV melanoma.

7. The method of any one of paragraphs 1-6, wherein the patient has an Eastern Cooperative Oncology Group (ECOG) performance status of 0 or 1.

8. The method of any one of paragraphs 1-7, wherein the patient has measurable disease as determined by Response Evaluation Criteria in Solid Tumors (RECIST) version 1.1.

9. The method of any one of paragraphs 1-8, wherein the patient's tumor infiltrating lymphocytes express LAG-3.

10. The method of paragraph 9, wherein greater than 1% of the patient's tumor infiltrating lymphocytes cells express LAG-3.

11. The method of paragraphs 9 or 10, wherein LAG-3 expression is determined from a tumor biopsy test tissue sample.

12. The method of any one of paragraphs 9-11, wherein the test tissue sample is a formalin-fixed paraffin embedded (FFPE) sample.

13. The method of any one of paragraphs 9-12, wherein LAG-3 expression is determined by measuring protein or RNA expression in the test tissue sample.

14. The method of any one of paragraphs 9-13, wherein LAG-3 expression is detected by an assay capable of detecting the level of LAG-3 protein in the test tissue sample.

15. The method of paragraph 14, wherein LAG-3 expression is detected by an immunohistochemistry assay.

16. The method of paragraph 15, wherein the immunohistochemistry assay is a monoplex assay.

17. The method of paragraph 15, wherein the immunohistochemistry assay is a multiplex assay.

18. The method of any one of paragraphs 15-17, wherein the immunohistochemistry assay comprises contacting the tumor sample with the 17B4, SP346, 11E3, 874501, or EPR4392(2) anti-human LAG-3 monoclonal antibody.

19. The method of any one of paragraphs 1-18, wherein the patient's tumor cells express PD-L1.

20. The method of paragraph 19, wherein greater than 1% of the patient's tumor cells express PD-L1.

21. The method of paragraphs 19 or 20, wherein PD-L1 expression is determined from a tumor biopsy test tissue sample.

22. The method of any one of paragraphs 19-21, wherein the test tissue sample is a formalin-fixed paraffin embedded (FFPE) sample.

23. The method of any one of paragraphs 19-22, wherein PD-L1 expression is determined by measuring protein or RNA expression in the test tissue sample.

24. The method of any one of paragraphs 19-23, wherein PD-L1 expression is detected by an assay capable of detecting the level of PD-L1 protein in the test tissue sample.

25. The method of paragraph 24, wherein PD-L1 expression is detected by an immunohistochemistry assay.

26. The method of paragraph 25, wherein the immunohistochemistry assay is a monoplex assay.

27. The method of paragraph 25, wherein the immunohistochemistry assay is a multiplex assay.

28. The method of any one of paragraphs 1-27, wherein the patient's tumor cells contain a BRAF V600 mutation.

29. The method of any one of paragraphs 1-28, wherein the LAG-3 antagonist is an anti-LAG-3 antibody.

30. The method of paragraph 29, wherein the anti-LAG-3 antibody is a full-length antibody.

31. The method of paragraph 30, wherein the antibody is a monoclonal, human, humanized, chimeric, or multispecific antibody.

32. The method of paragraph 31, wherein the multispecific antibody is a dual-affinity re-targeting antibody (DART), a DVD-Ig, or bispecific antibody.

33. The method of paragraph 29, wherein the antibody is a $F(ab')_2$ fragment, a Fab' fragment, a Fab fragment, a Fv fragment, a scFv fragment, a dsFv fragment, a dAb fragment, or a single chain binding polypeptide.

34. The method of paragraph 29, wherein the anti-LAG-3 antibody is BMS-986016, IMP731 (H5L7BW), MK-4280 (28G-10), REGN3767, GSK2831781, humanized BAP050, IMP-701 (LAG-525), aLAG3(0414), aLAG3(0416), Sym022, TSR-033, TSR-075, XmAb22841, MGD013, BI754111, FS118, P 13B02-30, or AVA-017.

35. The method of any of paragraphs 1-28, wherein the LAG-3 antagonist is IMP321.

36. The method of any of paragraphs 1-31, wherein the anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5.

37. The method of any of paragraphs 1-31 and 36, wherein the anti-LAG-3 antibody comprises

(a) a heavy chain variable region CDR1 comprising the sequence set forth in SEQ ID NO:7;
(b) a heavy chain variable region CDR2 comprising the sequence set forth in SEQ ID NO:8;
(c) a heavy chain variable region CDR3 comprising the sequence set forth in SEQ ID NO:9;
(d) a light chain variable region CDR1 comprising the sequence set forth in SEQ ID NO:10;
(e) a light chain variable region CDR2 comprising the sequence set forth in SEQ ID NO:11; and
(f) a light chain variable region CDR3 comprising the sequence set forth in SEQ ID NO:12.

38. The method of any of paragraphs 1-31, 36 and 37, wherein the anti-LAG-3 antibody comprises heavy and light chain variable regions comprising the sequences set forth in SEQ ID NOs:3 and 5, respectively.

39. The method of any of paragraphs 1-31 and 36-38, wherein the anti-LAG-3 antibody comprises heavy and light chains comprising the sequences set forth in SEQ ID NOs: 1 and 2, respectively.

40. The method of any of paragraphs 1-39, wherein the PD-1 pathway inhibitor is an anti-PD-1 or an anti-PD-L1 antibody.

41. The method of paragraph 40, wherein the anti-PD-1 antibody is selected from the group consisting of: nivolumab, pembrolizumab, pidilizumab, PDR001, MEDI0680, TSR-042, REGN2810, JS001, PF-06801591, BGB-A317, BI 754091, and SHR-1210.

42. The method of any of paragraphs 1-41, wherein the anti-PD-1 antibody comprises CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17.

43. The method of any of paragraphs 1-42, wherein the anti-PD-1 antibody comprises

(a) a heavy chain variable region CDR1 comprising the sequence set forth in SEQ ID NO:19;
(b) a heavy chain variable region CDR2 comprising the sequence set forth in SEQ ID NO:20;
(c) a heavy chain variable region CDR3 comprising the sequence set forth in SEQ ID NO:21;
(d) a light chain variable region CDR1 comprising the sequence set forth in SEQ ID NO:22;
(e) a light chain variable region CDR2 comprising the sequence set forth in SEQ ID NO:23; and
(f) a light chain variable region CDR3 comprising the sequence set forth in SEQ ID NO:24.

44. The method of any of paragraphs 1-43, wherein the anti-PD-1 antibody comprises heavy and light chain variable regions comprising the sequences set forth in SEQ ID NOs: 15 and 17, respectively.

45. The method of any of paragraphs 1-44, wherein the anti-PD-1 antibody comprises heavy and light chains comprising the sequences as set forth in SEQ ID NOs: 13 and 14, respectively.

46. The method of any of paragraphs 1-45, wherein a fixed dose combination of the anti-LAG-3 or LAG-3 antagonist and anti-PD-1 antibody are administered.

47. The method of any of paragraphs 1-46, wherein the method comprises at least one administration cycle, wherein the cycle is a period of four weeks, and wherein for each of the at least one cycle, one dose of the anti-LAG-3 antibody or antagonist is administered at a dose of 160 mg and one dose of the anti-PD-1 antibody are administered at a dose of 480 mg.

48. The method of any of paragraphs 1-47, wherein the anti-LAG-3 antibody or antagonist and anti-PD-1 antibody are formulated for intravenous administration.

49. The method of any of paragraphs 1-48, wherein the anti-LAG-3 antibody or antagonist and anti-PD-1 antibody are formulated together.

50. The method of any of paragraphs 1-48, wherein the anti-LAG-3 antibody or antagonist and anti-PD-1 antibody are formulated separately.

51. A method of inhibiting the growth of a metastatic melanoma tumor in a human patient, the method comprising administering to the patient an effective amount of each of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma.

52. A method of treating metastatic melanoma in a human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma.

53. A method of inhibiting the growth of an unresectable melanoma tumor in a human patient, the method comprising administering to the patient an effective amount of each of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma.

54. A method of treating unresectable melanoma in a human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma.

55. A method of inhibiting the growth of a metastatic melanoma tumor in a human patient, the method comprising administering to the patient an effective amount of each of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma; and wherein at least one dose of the anti-LAG-3 antibody is administered at a dose of 160 mg and at least one dose of the anti-PD-1 antibody are administered at a dose of 480 mg every four weeks.

56. A method of treating metastatic melanoma in a human patient, the method comprising administering to the patient an effective amount of :

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma; and wherein at least one dose of the anti-LAG-3 antibody is administered at a dose of 160 mg and at least one dose of the anti-PD-1 antibody is administered at a dose of 480 mg every four weeks.

57. A method of inhibiting the growth of an unresectable melanoma tumor in a human patient, the method comprising administering to the patient an effective amount of each of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

58. A method of treating unresectable melanoma in a human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

59. A method of inhibiting the growth of a metastatic melanoma tumor in an adolescent human patient, the method comprising administering to the patient an effective amount of each of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma; and wherein at least one dose of the anti-LAG-3 antibody is administered at a dose of 2 mg/kg and at least one dose of the anti-PD-1 antibody is administered at a dose of 6 mg/kg every four weeks.

60. A method of treating metastatic melanoma in an adolescent human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma; and wherein at least one dose of the anti-LAG-3 antibody is administered at a dose of 2 mg/kg and at least one dose of the anti-PD-1 antibody is administered at a dose of 6 mg/kg every four weeks.

61. A method of inhibiting the growth of an unresectable melanoma tumor in an adolescent human patient, the method comprising administering to the patient an effective amount of each of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma; and wherein at least one dose of the anti-LAG-3 antibody is administered at a dose of 2 mg/kg and at least one dose of the anti-PD-1 antibody is administered at a dose of 6 mg/kg every four weeks.

62. A method of treating unresectable melanoma in an adolescent human patient, the method comprising administering to the patient an effective amount of:

(a) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(b) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma; and wherein at least one dose of the anti-LAG-3 antibody is administered at a dose of 2 mg/kg and at least one dose of the anti-PD-1 antibody is administered at a dose of 6 mg/kg every four weeks.

63. The method of any one of paragraphs 59-62, wherein the patient is 12 or more years of age.

64. The method of paragraph 63, wherein the patient is between 12 and 17 years of age.

65. The method of any one of paragraphs 51-64, wherein the prior system treatment is adjuvant or neoadjuvant anti-PD-1 or anti-CTLA-4 therapy.

66. The method of any one of paragraphs 51-65, wherein the patient has histologically confirmed unresectable stage III or IV melanoma.

67. The method of any one of paragraphs 51-66, wherein the patient has an Eastern Cooperative Oncology Group (ECOG) performance status of 0 or 1 and/or a Lansky performance score of 80% or greater.

68. The method of any one of paragraphs 51-67, wherein the patient has measurable disease as determined by Response Evaluation Criteria in Solid Tumors (RECIST) version 1.1.

69. The method of any one of paragraphs 51-68, wherein the patient's tumor infiltrating lymphocytes cells express LAG-3.

70. The method of paragraph 69, wherein greater than 1% of the patient's tumor 71.
The method of paragraph 142, wherein LAG-3 expression is determined from a tumor biopsy test tissue sample.

71. The method of paragraph 69 or 70, wherein the test tissue sample is a formalin-fixed paraffin embedded (FFPE) sample.

72. The method of any one of paragraphs 69-71, wherein LAG-3 expression is determined by measuring protein or RNA expression in the test tissue sample.

73. The method of any one of paragraphs 69-72, wherein LAG-3 expression is detected by an assay capable of detecting the level of LAG-3 protein in the test tissue sample.

74. The method of paragraph 73, wherein LAG-3 expression is detected by an immunohistochemistry assay.

75. The method of paragraph 74, wherein the immunohistochemistry assay is a monoplex assay.

76. The method of paragraph 74, wherein the immunohistochemistry assay is a multiplex assay.

77. The method of any one of paragraphs 74-76, wherein the immunohistochemistry assay comprises contacting the tumor sample with the 17B4, SP346, 11E3, 874501, or EPR4392(2) anti-human LAG-3 monoclonal antibody associated tumor infiltrating lymphocytes express LAG-3.

78. The method of any one of paragraphs 51-77, wherein the patient's tumor cells express PD-L1.

79. The method of paragraph 78, wherein greater than 1% of the patient's tumor cells express PD-L1.

80. The method of paragraphs 78 or 79, wherein PD-L1 expression is determined from a tumor biopsy test tissue sample.

81. The method of any one of paragraphs 78-80, wherein the test tissue sample is a formalin-fixed paraffin embedded (FFPE) sample.

82. The method of any one of paragraphs 78-82, wherein PD-L1 expression is determined by measuring protein or RNA expression in the test tissue sample.

83. The method of paragraph 82, wherein PD-L1 expression is detected by an assay capable of detecting the level of PD-L1 protein in the test tissue sample.

84. The method of paragraph 83, wherein PD-L1 expression is detected by an immunohistochemistry assay.

85. The method of paragraph 84, wherein the immunohistochemistry assay is a monoplex assay.

86. The method of paragraph 84, wherein the immunohistochemistry assay is a multiplex assay.

87. The method of any one of paragraphs 51-86, wherein the patient's tumor cells contain a BRAF V600 mutation.

88. A method of inhibiting the growth of a metastatic melanoma tumor in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma.

89. A method of treating metastatic melanoma in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma.

90. A method of inhibiting the growth of an unresectable melanoma tumor in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma.

91. A method of treating unresectable melanoma in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma.

92. A method of inhibiting the growth of a metastatic melanoma tumor in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma; and wherein at least one dose of the anti-LAG-3 antibody is administered at a dose of 160 mg and at least one dose of the anti-PD-1 antibody are administered at a dose of 480 mg every four weeks.

93. A method of treating metastatic melanoma in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for metastatic melanoma; and wherein at least one dose of the anti-LAG-3 antibody is administered at a dose of 160 mg and at least one dose of the anti-PD-1 antibody is administered at a dose of 480 mg every four weeks.

94. A method of inhibiting the growth of an unresectable melanoma tumor in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

95. A method of treating unresectable melanoma in a human patient, the method comprising:

(a) determining expression of LAG-3 on the patient's tumor infiltrating lymphocytes, PD-L1 expression on the patient's tumor cells, and/or determining the presence of a BRAF V600 mutation in the patient's tumor cells; and

(b) administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior systemic treatment for unresectable melanoma; and wherein at least one dose of the anti-LAG-3 antibody is administered at a dose of 160 mg and at least one dose of the anti-PD-1 antibody is administered at a dose of 480 mg every four weeks.

96. A method of treating unresectable melanoma in a human patient, the method comprising administering to the patient an effective amount of each of:

(i) an anti-LAG-3 antibody comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5, and

(ii) an anti-PD-1 antibody comprising CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

wherein the patient has not received prior treatment for unresectable melanoma; and wherein at least one dose of the anti-LAG-3 antibody is administered at a dose of 160 mg and at least one dose of the anti-PD-1 antibody is administered at a dose of 480 mg every four weeks.

97. The method of paragraph 96, wherein the anti-LAG-3 antibody and the anti-PD-1 antibody are administered as a first-line treatment.

98. A method of treating unresectable melanoma in a human patient, the method comprising administering to the patient an effective amount of each of:

(i) an anti-LAG-3 antibody comprising (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO:11, and
(ii) an anti-PD-1 antibody comprising (a) a heavy chain variable region CDR1 comprising the sequence set forth in SEQ ID NO:19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24,

wherein the patient has not received prior treatment for unresectable melanoma; and wherein at least one dose of the anti-LAG-3 antibody is administered at a dose of 160 mg and at least one dose of the anti-PD-1 antibody is administered at a dose of 480 mg every four weeks.

99. The method of paragraph 96, wherein the anti-LAG-3 antibody and the anti-PD-1 antibody are administered as a first-line treatment.

100. The method of any of paragraphs 1-31 and 36-38 and 40-99, wherein the anti-LAG-3 antibody comprises heavy and light chains comprising the sequences set forth in SEQ ID NOs: 30 and 2, respectively.

101. A method of treating unresectable melanoma in a human patient, the method comprising administering to the patient an effective amount of each of relatlimab and nivolumab, wherein the patient has not received prior treatment for unresectable melanoma; and wherein at least one dose of the relatlimab is administered at a dose of 160 mg and at least one dose of the nivolumab is administered at a dose of 480 mg every four weeks.

102. A method of treating unresectable melanoma in a human patient, the method comprising administering to the patient an effective amount of each of:

(i) an anti-LAG-3 antibody comprising heavy and light chains comprising the sequences set forth in SEQ ID NOs: 30 and 2, respectively, and
(ii) an anti-PD-1 antibody comprising the sequences as set forth in SEQ ID NOs: 13 and 14, respectively,

wherein the patient has not received prior treatment for unresectable melanoma; and wherein at least one dose of the anti-LAG-3 antibody is administered at a dose of 160 mg and at least one dose of the anti-PD-1 antibody is administered at a dose of 480 mg every four weeks.

103. A method of treating metastatic melanoma in a human patient, the method comprising administering to the patient an effective amount of each of relatlimab and nivolumab, wherein the patient has not received prior treatment for metastatic melanoma; and wherein at least one dose of the relatlimab is administered at a dose of 160 mg and at least one dose of the nivolumab is administered at a dose of 480 mg every four weeks.

104. A method of treating metastatic melanoma in a human patient, the method comprising administering to the patient an effective amount of each of:

(i) an anti-LAG-3 antibody comprising heavy and light chains comprising the sequences set forth in SEQ ID NOs: 30 and 2, respectively, and
(ii) an anti-PD-1 antibody comprising the sequences as set forth in SEQ ID NOs: 13 and 14, respectively,

wherein the patient has not received prior treatment for metastatic melanoma; and wherein at least one dose of the anti-LAG-3 antibody is administered at a dose of 160 mg and at least one dose of the anti-PD-1 antibody is administered at a dose of 480 mg every four weeks.

105. A method of treating metastatic or unresectable melanoma in a human patient, the method comprising administering to the patient an effective amount of each of relatlimab and nivolumab, wherein the patient has not received prior treatment for metastatic or unresectable melanoma; and wherein at least one dose of relatlimab is administered at a dose of 160 mg and at least one dose of nivolumab is administered at a dose of 480 mg every four weeks.

106. A method of treating metastatic or unresectable melanoma in a human patient, the method comprising administering to the patient an effective amount of each of:

(i) an anti-LAG-3 antibody comprising heavy and light chains comprising the sequences set forth in SEQ ID NOs: 30 and 2, respectively, and
(ii) an anti-PD-1 antibody comprising the sequences as set forth in SEQ ID NOs: 13 and 14, respectively,

wherein the patient has not received prior treatment for metastatic or unresectable melanoma; and wherein at least one dose of the anti-LAG-3 antibody is administered at a dose of 160 mg and at least one dose of the anti-PD-1 antibody is administered at a dose of 480 mg every four weeks.

107. A product comprising relatlimab and nivolumab for combined use in treating metastatic or unresectable melanoma in a human patient that has not received prior treatment for metastatic or unresectable melanoma; wherein the treatment comprises administering at least one dose of relatlimab at a dose of 160 mg and at least one dose of nivolumab at a dose of 480 mg every four weeks.

108. A product comprising an anti-LAG-3 antibody comprising heavy and light chains comprising the sequences set forth in SEQ ID NOs: 30 and 2, respectively and an anti-PD-1 antibody comprising the sequences as set forth in SEQ ID NOs: 13 and 14, respectively for combined use in treating metastatic or unresectable melanoma in a human patient that has not received prior treatment for metastatic or unresectable melanoma; wherein the treatment comprises administering at least one dose of the anti-LAG-3 antibody at a dose of 160 mg and at least one dose of the anti-PD-1 antibody at a dose of 480 mg every four weeks.

109. A product comprising an anti-LAG-3 antibody comprising (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO:11, and an anti-PD-1 antibody comprising (a) a heavy chain variable region CDR1 comprising the sequence set forth in SEQ ID NO:19; (b) a heavy chain variable region CDR2 comprising the sequence set forth in SEQ ID NO:20; (c) a heavy chain variable region CDR3 comprising the sequence set forth in SEQ ID NO:21; (d) a light chain variable region CDR1 comprising the sequence set forth in SEQ ID NO:22; (e) a light chain variable region CDR2 comprising the sequence set forth in SEQ ID NO:23; and (f) a light chain variable region CDR3 comprising the sequence set forth in SEQ ID NO:24, for combined use in treating metastatic or unresectable melanoma in a human patient that has not received prior treatment for metastatic or unresectable melanoma; wherein the treatment comprises administering at least one dose of the anti-LAG-3 antibody at a dose of 160 mg and at least one dose of the anti-PD-1 antibody at a dose of 480 mg every four weeks.

## Claims

1. A combination comprising an anti-LAG-3 antibody and an anti-PD-1 antibody for use in a method of treating a metastatic melanoma tumor or an unresectable melanoma tumor in a human patient, the method comprising administering to the patient 160 mg of the anti-LAG-3 antibody and 480 mg of the anti-PD-1 antibody.

2. An anti-LAG-3 antibody for use in a method of treating a metastatic melanoma tumor or an unresectable melanoma tumor in a human patient, the method comprising administering to the patient 160 mg of the anti-LAG-3 antibody and 480 mg of an anti-PD-1 antibody.

3. An anti-PD-1 antibody for use in a method of treating a metastatic melanoma tumor or an unresectable melanoma tumor in a human patient, the method comprising administering to the patient 160 mg of an anti-LAG-3 antibody and 480 mg of the anti-PD-1 antibody.

4. The combination, anti-LAG-3 antibody, or anti-PD-1 antibody for use according to any one of claims 1 to 3, wherein the method comprises at least one administration cycle, wherein the cycle is a period of four weeks, and wherein for each of the at least one cycle, one dose of the anti-LAG-3 antibody is administered at a dose of 160 mg and one dose of the anti-PD-1 antibody is administered at a dose of 480 mg.

5. The combination, anti-LAG-3 antibody, or anti-PD-1 antibody for use according to any one of claims 1 to 4, wherein the patient has not received any prior systemic treatment for the metastatic melanoma or the unresectable melanoma.

6. The combination, anti-LAG-3 antibody, or anti-PD-1 antibody for use according to claim 5, wherein the patient:

a) has histologically confirmed unresectable stage III or IV melanoma; and/or

b) is 12 or more years of age; and/or

c) has an Eastern Cooperative Oncology Group (ECOG) performance status of 0 or 1 and/or a Lansky performance score of 80% or greater; and/or

d) has measurable disease as determined by Response Evaluation Criteria in Solid Tumors (RECIST) version 1.1.

7. The combination, anti-LAG-3 antibody, or anti-PD-1 antibody for use according to claim 5 or 6, wherein

a) greater than 1% of the patient's tumor infiltrating lymphocytes cells express LAG-3; and/or

b) wherein greater than 1% of the patient's tumor cells express PD-L1; and/or

c) wherein the patient's tumor cells contain a BRAF V600 mutation.

8. The combination, anti-LAG-3 antibody, or anti-PD-1 antibody for use according to any one of claims 5 to 7,

a) wherein the anti-LAG-3 antibody is a full-length antibody and/or wherein the anti-PD-1 antibody is a full-length antibody; and/or

b) wherein the anti-LAG-3 antibody is a monoclonal, human, humanized, chimeric, or multispecific antibody; and/or

c) wherein the anti-PD-1 antibody is a monoclonal, human, humanized, chimeric, or multispecific antibody; and/or

d) wherein the anti-LAG-3 antibody is a multispecific antibody which is a dual-affinity retargeting antibody (DART), a DVD-Ig, or a bispecific antibody; and/or

e) wherein the anti-PD-1 antibody is a bispecific antibody.

9. The combination, anti-LAG-3 antibody, or anti-PD-1 antibody for use according to any one of claims 5 to 7,

wherein the anti-LAG-3 antibody is a F(ab')2 fragment, a Fab' fragment, a Fab fragment, a Fv fragment, a scFv fragment, a dsFv fragment, a dAb fragment, or a single chain binding polypeptide; and/or

wherein the anti-PD-1 antibody is a F(ab')2 fragment, a Fab fragment, a Fv fragment, a scFv fragment, a Fd fragment, a dAb fragment, or a single chain binding polypeptide.

10. The combination, anti-LAG-3 antibody, or anti-PD-1 antibody for use according to any one of claims 5 to 7, wherein the anti-LAG-3 antibody comprises:

a) relatlimab, IMP731 (H5L7BW), MK-4280 (28G-10), REGN3767, GSK2831781, humanized BAP050, IMP-701 (LAG-525), aLAG3(0414), aLAG3(0416), Sym022, TSR-033, MGD013, BI754111, FS118, or AVA-017;

b) CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5;

c) heavy chain variable region CDR1, CDR2, CDR3 domains comprising the sequence set forth in SEQ ID NO:7, 8 and 9, respectively, and light chain variable region CDR1, CDR2, CDR3 domains comprising the sequence set forth in SEQ ID NO:10, 11 and 12, respectively;

d) heavy and light chain variable regions comprising the sequences set forth in SEQ ID NOs:3 and 5, respectively; or

e) heavy and light chains comprising the sequences set forth in SEQ ID NOs: 1 and 2, respectively.

11. The combination, anti-LAG-3 antibody, or anti-PD-1 antibody for use according to any one of claims 5 to 7, wherein the anti-PD-1 antibody comprises:

a) nivolumab, pembrolizumab, pidilizumab, PDR001, MED10680, TSR-042, REGN2810, JS001, PF-06801591, BGB-A317, BI 754091, or SHR-1210;

b) CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

c) heavy chain variable region CDR1, CDR2, and CDR3 domains comprising the sequence set forth in SEQ ID NO:19, 20 and 21, respectively, and light chain variable region CDR1, CDR2, and CDR3 domains comprising the sequence set forth in SEQ ID NO:22, 23 and 24, respectively;

d) heavy and light chain variable regions comprising the sequences set forth in SEQ ID NOs: 15 and 17, respectively; or
e) heavy and light chains comprising the sequences as set forth in SEQ ID NOs: 13 and 14, respectively.

12. The combination, anti-LAG-3 antibody, or anti-PD-1 antibody for use according to any one of claims 5 to 11,

wherein the anti-LAG-3 antibody and anti-PD-1 antibody are formulated for parenteral administration,
optionally wherein the anti-LAG-3 antibody and anti-PD-1 antibody are formulated for intravenous, intraperitoneal, intramuscular, intrathecal, or subcutaneous administration; or
wherein the anti-LAG-3 antibody and anti-PD-1 antibody are formulated together, or
wherein the anti-LAG-3 antibody and anti-PD-1 antibody are formulated separately.

13. The combination, anti-LAG-3 antibody, or anti-PD-1 antibody for use according to any one of claims 5 to 12, the method comprising:

a) determining expression of LAG-3 on the patient's tumor infiltrating lymphocytes, PDL1 expression on the patient's tumor cells, and/or determining the presence of a BRAF V600 mutation in the patient's tumor cells; and
b) administering to the patient the anti-LAG-3 antibody and anti-PD-1 antibody, wherein:

i) the anti-LAG-3 antibody comprises CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5; and
ii) the anti-PD-1 antibody comprises CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17.

14. The combination, anti-LAG-3 antibody, or anti-PD-1 antibody for use according to any one of claims 1 to 12, the method comprising:

a) determining the level of LAG-3 expression or the level of LAG-3 and PD-L1 expression in a tumor sample, optionally determining the presence of tumor cells expressing the BRAF V600 mutation; and
b) administering to the patient the anti-LAG-3 antibody and anti-PD-1 antibody.

15. The combination, anti-LAG-3 antibody, or anti-PD-1 antibody for use according to any one of claims 1 to 14, wherein the method further comprises the use of an anti-CTLA-4 antibody.

16. The combination, anti-LAG-3 antibody, or anti-PD-1 antibody for use according to claim 15, wherein the anti-CTLA-4 antibody is ipilimumab (YERVOY), tremelimumab (ticilimumab; CP-675,206), AGEN-1884, or ATOR-1015.

17. The combination, anti-LAG-3 antibody, or anti-PD-1 antibody for use according to any one of claims 1 to 4 or 14 to 16, wherein the anti-LAG-3 antibody comprises:

a) relatlimab, IMP731 (H5L7BW), MK-4280 (28G-10), REGN3767, GSK2831781, humanized BAP050, IMP-701 (LAG-525), aLAG3(0414), aLAG3(0416), Sym022, TSR-033, MGD013, B754111, FS118, or AVA-017;
b) CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5;
c) heavy chain variable region CDR1, CDR2, CDR3 domains comprising the sequence set forth in SEQ ID NO:7, 8 and 9, respectively, and light chain variable region CDR1, CDR2, CDR3 domains comprising the sequence set forth in SEQ ID NO:10, 11 and 12, respectively;
d) heavy and light chain variable regions comprising the sequences set forth in SEQ ID NOs:3 and 5, respectively; or
e) relatlimab comprising heavy and light chains comprising the sequences set forth in SEQ ID NOs: 1 and 2, respectively.

18. The combination, anti-LAG-3 antibody, or anti-PD-1 antibody for use according to any one of claims 1 to 4, 14 to 17, wherein the anti-PD-1 antibody comprises:

a) nivolumab, pembrolizumab, pidilizumab, PDR001, MED10680, TSR-042, REGN2810, JS001, BGB-A317, or SHR-1210;

b) CDR1, CDR2 and CDR3 domains of the heavy chain variable region having the sequence set forth in SEQ ID NO: 15, and CDR1, CDR2 and CDR3 domains of the light chain variable region having the sequence set forth in SEQ ID NO:17;

c) heavy chain variable region CDR1, CDR2, and CDR3 domains comprising the sequence set forth in SEQ ID NO:19, 20 and 21, respectively, and light chain variable region CDR1, CDR2, and CDR3 domains comprising the sequence set forth in SEQ ID NO:22, 23 and 24, respectively; or

d) heavy and light chain variable regions comprising the sequences set forth in SEQ ID NOs: 15 and 17, respectively.

**EP 4 663 244 A2**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62748089
- US 8008449 B
- US 7943743 B
- US 2016029878 W
- US 1348999 W
- US 20110150892 A1
- US 2011007023 A
- WO 2016028672 A
- WO 2018185046 A
- WO 2018185043 A
- WO 2017019894 A
- WO 2017106129 A
- WO 2017062888 A
- WO 2009044273 A
- WO 2018069500 A
- WO 2016126858 A
- WO 2014179664 A
- WO 2016200782 A
- WO 2015200119 A
- WO 2017019846 A
- WO 2017198741 A
- WO 2017220555 A
- WO 2017220569 A
- WO 2018071500 A
- WO 2017015560 A
- WO 2017025498 A
- WO 2017087589 A
- WO 2017087901 A
- WO 2018083087 A
- WO 2017149143 A
- WO 2017219995 A
- US 20170260271 A
- WO 2017086367 A
- WO 2017086419 A
- WO 2018034227 A
- WO 2014140180 A
- WO 2006121168 A
- WO 2008156712 A
- WO 2012145493 A
- WO 2009014708 A
- WO 03099196 A
- WO 2009114335 A
- WO 2011161699 A
- US 6808710 B
- US 7488802 B
- US 8168757 B
- US 8354509 B
- US 20160272708 A
- WO 2015112900 A
- WO 2015112800 A
- WO 2014206107 A
- WO 201535606 A
- WO 2015085847 A
- WO 2017020291 A
- WO 2017020858 A
- WO 2016197367 A
- WO 2017024515 A
- WO 2017025051 A
- WO 2017123557 A
- WO 2016106159 A
- WO 2014194302 A
- WO 2017040790 A
- WO 2017133540 A
- WO 2017132827 A
- WO 2017024465 A
- WO 2017025016 A
- WO 2017106061 A
- WO 201719846 A
- WO 2017132825 A
- US 20150079109 A
- US 8900587 B
- US 8779105 B
- WO 2013173223 A
- US 9580507 B
- US 8217149 B
- WO 2011066389 A
- WO 2013079174 A
- WO 2013181634 A
- WO 2016149201 A
- WO 2017034916 A

**Non-patent literature cited in the description**

- **TRIEBEL F et al.** *J. Exp. Med.*, 1990, vol. 171, 1393-1405
- **WORKMAN C J et al.** *J. Immunol.*, 2009, vol. 182 (4), 1885-91
- **KEIR M E et al.** *Annu Rev Immunol*, 2008, vol. 26, 677-704
- **DONG H et al.** *Nat Med.*, 1999, vol. 5, 1365-1369

48

- **TERME M et al.** *Cancer Res*, 2011, vol. 71, 5393-5399
- **PARDOLL D M.** *Nat Rev Cancer*, 2012, vol. 12, 252-264
- **NISHIMURA H et al.** *Immunity*, 1999, vol. 11, 141-151
- **NISHIMURA H et al.** *Science*, 2001, vol. 291, 319-322
- **DONG H et al.** *Nat Med*, 2002, vol. 8, 793-800
- **TOPALIAN S L et al.** *NEJM*, 2012, vol. 366 (26), 2443-2454
- **HAMID O et al.** *NEJM*, 2013, vol. 369, 134-144
- **WARD et al.** *Nature*, 1989, vol. 341, 544-46
- **BIRD et al.** *Science*, 1988, vol. 242, 423-426
- **HUSTON et al.** *Proc. Natl. Acad. Sci. USA*, 1988, vol. 85, 5879-5883
- **OKAZAKI et al.** *Curr. Opin. Immunol.*, 2002, vol. 14, 391779-82
- **BENNETT et al.** *J Immunol*, 2003, vol. 170, 711-8
- **HUTLOFF et al.** *Nature*, 1999, vol. 397, 263-266
- **HANSEN et al.** *Immunogenics*, 1980, vol. 10, 247-260
- **ISHIDA et al.** *EMBO J*, 1992, vol. 11, 3887-95
- **AGATA et al.** *Int Immunol*, 1996, vol. 8, 765-72
- **THOMAS, M. L.** *J Exp Med*, 1995, vol. 181, 1953-6
- **VIVIER, E ; DAERON, M**. *Immunol Today*, 1997, vol. 18, 286-91
- **FREEMAN et al.** *J Exp Med*, 2000, vol. 192, 1027-34
- **LATCHMAN et al.** *Nat Immunol*, 2001, vol. 2, 261-8
- **CARTER et al.** *Eur J Immunol*, 2002, vol. 32, 634-43
- **DONG et al.** *Nat. Med.*, 2002, vol. 8, 787-9
- **DONG et al.** *J. Mol. Med.*, 2003, vol. 81, 281-7
- **BLANK et al.** *Cancer Immunol. Immunother.*, 2005, vol. 54, 307-314
- **KONISHI et al.** *Clin. Cancer Res.*, 2004, vol. 10, 5094-100
- **IWAI et al.** *Proc. Nat'1. Acad. Sci. USA*, 2002, vol. 99, 12293-7
- **BROWN et al.** *J. Immunol.*, 2003, vol. 170, 1257-66
- **NISHIMURA et al.** *Immunity*, 1999, vol. 11, 141-51
- **NISHIMURA et al.** *Science*, 2001, vol. 291, 319-22
- **SALAMA et al.** *J Exp Med*, 2003, vol. 198, 71-78
- **PROKUNINA ; ALARCON-RIQUELME**. *Hum Mol Genet*, 2004, vol. 13, 143
- **NIELSEN et al.** *Lupus*, 2004, vol. 13, 510
- **OKAZAKI et al.** *PNAS*, 2001, vol. 98, 13866-71
- Concise Dictionary of Biomedicine and Molecular Biology. CRC Press, 2002
- The Dictionary of Cell and Molecular Biology. Academic Press, 2013
- Oxford Dictionary Of Biochemistry And Molecular Biology. Oxford University Press, 2006
- **CONDEELIS ; WEISSLEDER**. *Cold Spring Harb. Perspect. Biol.*, 2010, vol. 2 (12), a003848
- **MCCABE ; WU**. *Cancer Biother. Radiopharm.*, 2010, vol. 25 (3), 253-61
- **OLAFSEN et al.** *Protein Eng. Des. Sel.*, 2010, vol. 23 (4), 243-9
- *Journal for ImmunoTherapy of Cancer*, 2016, vol. 4
- **J. MATSUZAKI et al.** *PNAS*, 2010, vol. 107, 7875
- **WANG et al.** *Cancer Immunol Res.*, 2014, vol. 2 (9), 846-56
- **SI-YANG LIU et al.** *J. Hematol. Oncol.*, 2017, vol. 10, 136
- **BRAHMER et al.** *N Engl J Med*, 2012, vol. 366, 2455-65
- **TOPALIAN et al.** *N Engl J Med*, 2012, vol. 366, 2443-54
- **HERBST et al.** *J Clin Oncol*, 2013, vol. 31, 3000
- **ZHANG et al.** *Cell Discov.*, March 2017, vol. 7, 3
- **GORELIK et al.** *AACR:Abstract 4606*, April 2016
- American Joint Committee on Cancer